(19) Europäisches Patentamt
European Patent Office
Office européen des brevets

(11) Veröffentlichungsnummer: **0 023 192**
**B1**

(12) # EUROPÄISCHE PATENTSCHRIFT

(45) Veröffentlichungstag der Patentschrift: **28.03.84**

(51) Int. Cl.³: **C 07 C 103/52,** A 61 K 37/02

(21) Anmeldenummer: **80810216.4**

(22) Anmeldetag: **30.06.80**

(54) **Cyclopeptide und pharmazeutische Präparate davon, sowie Verfahren zu ihrer Herstellung und deren Anwendung.**

(30) Priorität: **04.07.79 CH 6243/79**
**12.02.80 CH 1135/80**

(43) Veröffentlichungstag der Anmeldung: **28.01.81 Patentblatt 81/4**

(45) Bekanntmachung des Hinweises auf die Patenterteilung: **28.03.84 Patentblatt 84/13**

(84) Benannte Vertragsstaaten: **AT BE CH DE FR GB IT LI LU NL SE**

(56) Entgegenhaltungen: **EP - A - 0 001 295**

(73) Patentinhaber: **CIBA-GEIGY AG, Postfach, CH-4002 Basel (CH)**

(72) Erfinder: **Rink, Hans, Rebenstrasse 10, CH-4125 Riehen (CH)**
Erfinder: **Sieber, Peter, Bachmattweg 10, CH-4153 Reinach (CH)**
Erfinder: **Kamber, Bruno, Dr., Sonnenweg 9, CH-4144 Arlesheim (CH)**

**EP 0 023 192 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Cyclopeptide und pharmazeutische Präparate davon, sowie Verfahren zu ihrer Herstellung und deren Anwendung

Die Erfindung betrifft neue Cyclopeptide vom Somatostatin-Typ und ihre Herstellungsverfahren, sowie pharmazeutische Präparate enthaltend diese Verbindungen und Verwendung dieser Verbindungen bzw. Präparate zu therapeutischen Zwekken.

Die Erfindung betrifft insbesondere Cyclopeptide, welche die wesentlichsten Merkmale des Somatostatins, wie die Teilsequenz der Aminosäuren 5–11, oder eine äquivalente Sequenz enthalten, dabei aber schwefelfrei sind. Die erfindungsgemässen Somatostatin-analogen Cyclopeptide umfassen cyclische Octapeptide der Formel

└Asn–Phe–Phe–trp–Lys–Thr–Phe–Gaba(Ar)┘ (I)
5 6 7 8 9 10 11 12

worin trp für L–Trp oder D–Trp, in welchen der Benzolring durch ein Halogenatom substituiert sein kann, und Gaba(Ar) für den Rest einer durch ein cyclisches Hydrocarbyl substituierten $\gamma$-Aminobuttersäure steht, sowie Säureadditionssalze und Komplexe davon.

Das im Benzolring des $trp^8$-Rests gegebenenfalls vorhandene Halogenatom ist insbesondere ein Chlor- oder Fluoratom, das sich vorzugsweise in der 5-Stellung befindet; als trp sind besonders bevorzugt 5-F–D–trp (5-Fluor-D-tryptophyl) und vor allem D–trp.

Der als Gaba(Ar) bezeichnete Rest ist näher definiert durch die Formel

$$\begin{array}{c} \quad R_\gamma \quad R_\beta \quad R_\alpha \\ -NH-CH-CH-CH-CO- \end{array}$$

worin jeweils eines der Symbole $R_\alpha$, $R_\beta$ und $R_\gamma$ ein unsubstituierter oder substituierter cyclischer Hydrocarbylrest Ar ist und die übrigen zwei für Wasserstoff stehen. Die dem Rest Gaba(Ar) entsprechende Säure hat die Kurzbezeichnung H–Gaba(Ar)–OH.

Der cyclische Hydrocarbylrest Ar ist insbesondere ein mono-, di- oder polycyclischer Cycloalkylrest oder ein entsprechender, mindestens einen aromatischen Ring enthaltender Arylrest mit höchstens 18, vorzugsweise höchstens 12 Ringkohlenstoffatomen. Unter den Cycloalkylresten sind bevorzugt solche mit 3- bis 8-, und vor allem 5- und/oder 6-gliedrigen Ringen, wie beispielsweise Cyclopropyl, Cyclobutyl, Cycloheptyl, Cyclooctyl und ganz besonders Cyclopentyl und Cyclohexyl, ferner auch 1-Bicyclo[2,2,2]octyl, 2-Bicyclo[2,2,2]octyl, 2-Bicyclo[2,2,1]heptyl, 1- oder 2-Adamantyl, und 1- oder 2-Perhydronaphthyl, d.h. Bicyclo[4,4,0]decyl. Ein Arylrest ist in erster Linie ein Naphthyl, wie 1- oder 2-Naphthyl, ein entsprechendes teilweise hydriertes Naphthyl, wie insbesondere 1-, 2-, 5- oder 6-(1,2,3,4-Tetrahydronaphthyl), Phenyl, ein Anthryl, ein Fluorenyl und Azulenyl. Alle diese cyclischen Hydrocarbylreste können einen oder mehrere niederaliphatische Hydrocarbylreste, insbesondere Alkylreste mit höchstens 4 Kohlenstoffatomen, z.B. Methyl, Äthyl, Propyl, Isopropyl oder ein Butyl, und/oder weitere cyclische, insbesondere monocyclische Hydrocarbylreste, wie die oben definierten, tragen, wobei die Gesamtzahl der Kohlenstoffatome höchstens 18 Kohlenstoffatome beträgt. Als solche cyclischen Hydrocarbylreste sind beispielsweise 4,4-Dimethylcyclohexyl, ein Tolyl, wie 2-, 3- oder 4-Tolyl und ein Biphenylyl, z.B. 4-Biphenylyl, zu nennen.

In ihrem aromatischen Teil können die cyclischen Hydrocarbylreste durch ein, zwei oder mehrere, gleiche oder verschiedene Substituenten substituiert sein, wie Halogen, z.B. Chlor, Brom, Jod und insbesondere Fluor, Niederalkoxy, insbesondere ein solches, das sich von einem der obengenannten Niederalkylreste mit höchstens 4 Kohlenstoffatomen ableitet, darunter vor allem Methoxy, ferner auch Nitro und Amino, insbesondere primäres Amino, Diniederalkylamino und Acylamino, wie Niederalkanoylamino, z.B. Acetamino, sowie Phenoxyreste, die gegebenenfalls einen bis drei dieser Substituenten tragen können. Besonders bevorzugt sind durch die genannten Substituenten substituierte Phenylreste, wie 4-Fluorphenyl, 4-Chlorphenyl, 4-Nitrophenyl und insbesondere 3-Phenoxyphenyl, sowie auch Naphthylreste, z.B. 4-Chlor-1-naphthyl.

Der Rest Ar befindet sich in der $\alpha$-, $\gamma$- oder vorzugsweise $\beta$-Stellung der Kette der $\gamma$-Aminobuttersäure; demnach leiten sich besonders bevorzugte Reste der Formel Gaba(Ar) von den folgenden Buttersäuren ab: 4-Amino-3-cyclohexyl-, 4-Amino-3-phenyl-, 4-Amino-3-(4-fluorphenyl)-, 4-Amino-3-(4-nitrophenyl)-, 4-Amino-3-(4-chlor-1-naphthyl)-, 4-Amino-3-(2-naphthyl)- und vor allem 4-Amino-3-(1-naphthyl)- und 4-Amino-3-(3-phenoxyphenyl)-buttersäure.

Als Resultat der Substitution mit dem Rest Ar entsteht am entsprechenden Kohlenstoffatom der Säurekette ein Asymmetriezentrum, welches das Vorliegen von jeweils zwei Diastereomeren des erfindungsgemässen Cyclopeptids zur Folge hat, die gewünschtenfalls voneinander getrennt, oder aber gemeinsam als Diastereomerengemisch für dieselben Verwendungszwecke eingesetzt werden können. Im allgemeinen ist jeweils das als Isomer B bezeichnete Diastereomere bevorzugt, d.h. jenes, welches sich vom hydrophileren Diastereomeren bei der Gegenstromverteilung von entsprechenden geschützten Zwischenprodukten der Formel II (siehe weiter unten) ableitet.

Besonders bevorzugte erfindungsgemässe cyclische Octapeptide der Formel I sind die folgenden: [D–$Trp^8$; $\beta$-Cyclohexyl-$Gaba^{12}$]-cyclo-somatostatin(5–12)-octapeptid der Formel

└Asn–Phe–Phe–[D–trp]–Lys–Thr–Phe–($\beta$-Cyclohexyl)Gaba┘ (IA),

[D-Trp$^8$; β-Phenyl-Gaba$^{12}$]-cyclo-somatostatin-(5–12)-octapeptid der Formel

Asn–Phe–Phe–[D–trp]–Lys–Thr–Phe–
(β-Phenyl)Gaba (IB),

[5-F–D–Trp$^8$; β-(1-Naphthyl)-Gaba$^{12}$]-cyclo-somatostatin(5–12)-octapeptid der Formel

Asn–Phe–Phe–[5-F–D–trp]–Lys–Thr–Phe–
[β–(1-naphthyl)]Gaba (IC),

und vor allem [D–Trp$^8$; β-(1-Naphthyl)-Gaba$^{12}$]-cyclo-somatostatin(5–12)-octapeptid der Formel

Asn–Phe–Phe–[D–trp]–Lys–Thr–Phe–
5 6 7 8 9 10 11
[β(1-Naphthyl)]Gaba (ID);
12

und [D–Trp$^8$; β–(3-Phenoxyphenyl)-Gaba$^{12}$]-cyclo-somatostatin(5–12)-octapeptid der Formel

Asn–Phe–Phe–[D–trp]–Lys–Thr–Phe–
[β–(3-Phenoxyphenyl)]Gaba (IE)

jede dieser genannten Verbindungen kann als ein Diastereomerengemisch abgeleitet von einem racemischen [d,l-Gaba(Ar)]-Rest, oder aber als ein individuelles Diastereomeres, z.B. ein in den nachstehenden Beispielen beschriebenes und durch physikalische Daten charakterisiertes, abgeleitet von nur einer der optisch isomeren Formen des [Gaba(Ar)]-Rests, vorliegen. Besonders bevorzugt ist jeweils das Isomere B der oben hervorgehobenen Verbindungen.

Alle oben allgemein oder als bevorzugt charakterisierten Somatostatin-Analogen der Formel I können auch in Form von Säureadditionssalzen oder Komplexen vorliegen. Als Säureadditionssalze kommen besonders physiologisch verträgliche Salze mit üblichen therapeutisch anwendbaren Säuren in Betracht; von den anorganischen Säuren sind die Halogenwasserstoffsäuren, wie die Chlorwasserstoffsäure, aber auch Schwefelsäure und Phosphor- bzw. Pyrophosphorsäure zu nennen, von den organischen Säuren sind es in erster Linie die Sulfonsäuren, wie die Benzol- oder p-Toluol-Sulfonsäure, oder Niederalkansulfonsäuren, wie Methansulfonsäure, ferner auch Carbonsäuren, wie Essigsäure, Milchsäure, Palmitin- und Stearinsäure, Äpfelsäure, Weinsäure, Ascorbinsäure und Zitronensäure.

Unter Komplexen sind die in ihrer Struktur noch nicht völlig abgeklärten Verbindungen zu verstehen, die beim Zusatz gewisser anorganischer oder organischer Stoffe zu Peptiden entstehen und diesen eine verlängerte Wirkung verleihen. Solche Stoffe sind beispielsweise beschrieben für ACTH und andere adrenocorticotrop wirksame Peptide. Zu nennen sind z.B. anorganische Verbindungen, die sich von Metallen wie Calcium, Magnesium, Aluminium, Cobalt und insbesondere von Zink ableiten, vor allem schwerlösliche Salze, wie Phosphate, Pyrophosphate und Polyphosphate, sowie Hydroxyde dieser Metalle, ferner Alkalimetallpolyphosphate, z.B. Calgon N®, Calgon 322® oder Calgon 188®. Organische Stoffe, die eine Verlängerung der Wirkung hervorrufen, sind beispielsweise nicht antigene Gelatine-Arten, z.B. Polyoxygelatine, Polyvinylpyrrolidon und Carboxymethylcellulose, ferner Sulfonsäure- oder Phosphorsäureester von Alginsäure, Dextran, Polyphenolen und Polyalkoholen, vor allem Polyphloretinphosphat und Phytinsäure, sowie Polymerisate und Copolymerisate von basischen oder vor allem sauren Aminosäuren, z.B. Protamin oder Polyglutaminsäure.

Wenn nicht anders angegeben, beziehen sich die Kurzbezeichnungen der Aminosäurereste auf Reste der α-Aminosäuren der L-Reihe, welche in der Natur vorkommen.

Wenn nicht anders angegeben, bezeichnet der Begriff «nieder», wo immer er im Zusammenhang mit einem organischen Rest oder einer Verbindung vorkommt, einen solchen Rest oder eine solche Verbindung mit höchstens 7, vorzugsweise aber mit höchstens 4 Kohlenstoffatomen.

Die neuen erfindungsgemässen Cyclopeptide weisen eine physiologische Wirkung auf, die im Grundcharakter der Wirkung des Somatostatins ähnlich ist. Sie können deshalb in ähnlichen therapeutischen Indikationen wie dieses, z.B. insbesondere zur Behandlung von Funktionsstörungen, in denen die Sekretion des Wachstumshormons oder Glucagons übernormal hoch ist, wie bei Acromegalie oder Diabetes, mit Vorteil angewendet werden. Indem sie überdies auch Blutverluste im gastrointestinalen Trakt hemmen, können sie auch in diesem Indikationsbereich mit Erfolg eingesetzt werden.

Somatostatin, ein cyclisches Tetradecapeptid der Formel

H–Ala–Gly–Cys–Lys–Asn–Phe–Phe–Trp–Lys–
1 2 3 4 5 6 7 8 9
Thr–Phe–Thr–Ser–Cys–OH
10 11 12 13 14

[Science 179,77 (1973)] hemmt bekanntlich die hypophysengesteuerte Sekretion des Wachstumshormons (Somatotropin). Ausserdem hemmt es die sekretorische Aktivität des endocrinen Pancreas, wie die Sekretion von Insulin und Glucagon. Diese wertvollen Eigenschaften können beim Somatostatin selber nicht zur vollen praktischen Anwendung gelangen, da diese Verbindung eine zu kurze Wirkungsdauer aufweist. Zudem ist es oft von Vorteil, wenn der Wirkstoff seine hemmende Wirkung hauptsächlich auf eine der beiden Drüsen ausübt, wogegen die andere Drüse möglichst wenig beeinflusst werden sollte. (In den meisten Fällen ist die Hemmung der hypophysären Sekretion, d.h. die der Wachstumshormon-Ausschüttung, weniger gewünscht.) Deshalb wird angestrebt, durch Modifizierung der Grundsequenz, insbesondere durch Auslassen einzelner ursprünglicher Aminosäuren und/oder deren Austausch gegen andere, oft auch «unnatürliche» Aminosäuren, eine Dissoziation der Hemmwirkungen sowie eine möglichst lange Wirkungsdauer zu erlangen. So wurde z.B. gefunden, dass besonders vorteilhafte physiologische Eigenschaften dieser Art bei Cyclopeptiden vom Typ

$$\overline{\text{Asn–Phe–Phe–[D–trp]–Lys–Thr–Phe–[Gaba]}_p}$$

5 6 7 8 9 10 11 (M)

worin Gaba den Rest der $\gamma$-Aminobuttersäure und p die Zahl 0, 1 oder 2 darstellt, und weiteren nahe verwandten Verbindungen auftreten, vgl. unsere Europäische Patentanmeldung Nr. 78 100 994.9, veröffentlicht unter Nr. 0 001 295. – Als ein typisches Merkmal dieser Verbindungen kommt das Vorhandensein einer geraden Kohlenstoffkette vor, welche die ursprüngliche Gruppierung $-CH_2-S-S-CH_2-$ im Cystinrest von Somatostatin simulieren soll.

Überraschenderweise wurde nun festgestellt, dass durch eine ganz ungewöhnliche Modifizierung dieses einfachen, jeweils unsubstituierten Kettenabschnittes, d.h. durch die Substitution der $\gamma$-Aminosäure durch einen cyclischen Hydrocarbylrest, die gewünschte Aktivität nicht nur erhalten bleibt, sondern noch weiter gesteigert wird. So weisen die erfindungsgemässen Verbindungen der Formel I gegenüber Somatostatin sowohl eine erhöhte Hemmung der Insulin- und Glucagon-Sekretion wie auch eine wesentliche Verlängerung der Wirkungsdauer auf. Beispielsweise wurde bei der Bestimmung der Insulin- und Glucagon-Ausschüttung am isolierten perfundierten Rattenpankreas [G.M. Grodsky und R.E. Fanska in «Methods in Enzymology» 39, Part D, Seite 364; (J.G. Hardman und B.W. O'Malley, Editors; Academic Press, New York, 1975)] gefunden, dass das [D–trp$^8$; $\beta$-(1-Naphthyl)Gaba$^{12}$]-cyclo-somatostatin(5–12)-octapeptid (Formel ID) als Diastereomerengemisch eine etwa 10fache, als das isolierte Isomere B sogar eine etwa 20fache Hemmwirkung verglichen mit [D–trp$^8$; Gaba$^{12}$]-cyclo-somatostatin-(5–12)-octapeptid (Formel M, p = I) aufweist.

Die erfindungsgemässen Verbindungen können nach an sich bekannten Methoden hergestellt werden, indem man ein lineares Peptid der Formel

H–[II']-C (III)

worin II' einen der nachstehend definierten Formel II entsprechenden Rest darstellt, in welchem die amidische Bindung zwischen zwei beliebigen benachbarten Aminosäureresten des Peptidringes unterbrochen ist, und C für eine freie Hydroxylgruppe, eine durch eine Aktivierungsgruppe abgewandelte Hydroxylgruppe oder die Hydrazinogruppe $-NH-NH_2$ steht, cyclisiert und in einer erhaltenen Verbindung der Formel

$$\overline{\text{Asn–Phe–Phe–trp–Lys(A)–Thr(B)–Phe–Gaba(Ar)}}$$ (II)

worin Gaba(Ar) und trp die obgenannten Bedeutungen haben, A eine $\varepsilon$-Aminoschutzgruppe oder Wasserstoff und B eine Hydroxylschutzgruppe oder Wasserstoff bedeutet, wobei nur eines der Symbole A und B für Wasserstoff stehen kann, die Schutzgruppe(n) abspaltet.

Als $\varepsilon$-Amino-Schutzgruppen können alle in der Peptid-Chemie üblichen Amino-Schutzgruppen verwendet werden, wie sie in den entsprechenden Nachschlagewerken, z.B. in Houben-Weyl: Methoden der organischen Chemie; 4. Auflage, Band 15/I, E. Wünsch (Herausgeber): Synthese von Peptiden. (Georg Thieme Verlag, Stuttgart; 1974) zusammenfassend beschrieben werden. Bevorzugt sind acidolytisch abspaltbare Gruppen, wie in erster Linie die tert.-Butoxycarbonylgruppe und analoge Gruppen, z.B. die
tert.-Amyloxycarbonyl-, Isopropyloxycarbonyl-,
Diisopropylmethoxycarbonyl-,
Allyloxycarbonyl-, Cyclopentyloxycarbonyl-,
Cyclohexyloxycarbonyl-,
d-Isobornyloxycarbonyl- und
Adamantyloxycarbonylgruppe,
sowie auch Gruppen des Aralkyl-Typs, wie Benzhydryl und Triphenylmethyl (Trityl), oder bestimmte Aralkoxycarbonyl-Gruppen des 2-(p-Biphenylyl)-2-propyloxycarbonyl-Typs, die in der Schweizer Patentschrift 509 266 beschrieben sind.

Ferner kann man aber auch reduktiv oder basisch abspaltbare Amino-Schutzgruppen verwenden, z.B. insbesondere die Benzyloxycarbonyl-Gruppe und Benzyloxcarbonyl-Gruppen, die im aromatischen Teil durch Halogenatome, Nitrogruppen, Niederalkoxygruppen und/oder Niederalkylreste substituiert sind, wie die p-Chlor- und p-Brombenzyloxycarbonyl-, p-Nitrobenzyloxycarbonyl-, p-Methoxybenzyloxycarbonyl-, p-Tolyloxycarbonyl-Gruppe, oder aber die Isonicotinyloxycarbonylgruppe, weiter auch Acylgruppen, wie p-Toluolsulfonyl, Benzolsulfonyl, o-Nitrobenzolsulfenyl bzw. auch Formyl, Trifluoracetyl oder Phthaloyl.

Eine besonders vorteilhafte $\varepsilon$-Amino-Schutzgruppe ist eine Äthoxycarbonylgruppe, die in $\beta$-Stellung eine mit drei Kohlenwasserstoffresten substituierte Silylgruppe, wie Triphenylsilyl-, Dimethylbutyl-silyl- oder vor allem Trimethylsilylgruppe, trägt. Eine solche $\beta$-(Trihydrocarbylsilyl)-äthoxycarbonylgruppe, wie eine $\beta$-(Triniederalkylsilyl)-äthoxycarbonyl- z.B. insbesondere die $\beta$-(Trimethylsilyl)-äthoxycarbonylgruppe, bildet mit der zu schützenden $\varepsilon$-Aminogruppe eine entsprechende $\beta$-Trihydrocarbylsilyl-äthoxycarbonylaminogruppe (z.B. die $\beta$-Trimethylsilyläthoxycarbonylaminogruppe), welche gegen die Bedingungen der sauren Hydrolyse und der Hydrogenolyse beständig ist, aber unter ganz spezifischen, sehr milden Bedingungen sich durch Einwirkung von Fluoridionen abspalten lässt. In dieser Beziehung verhält sie sich analog wie die weiter unten als Carboxyl-Schutzgruppe beschriebene $\beta$-Silyläthylestergruppe. (Diese Ähnlichkeit muss bei der Synthese besonders berücksichtigt werden: bis auf Einzelfälle schliesst die Anwendung einer dieser Schutzgruppen die gleichzeitige Anwendung der anderen Schutzgruppe aus.) Weitere Einzelheiten sind weiter unten beim Schutz der Carboxylgruppe durch β-Silyläthylester angegeben.

Als Hydroxylschutz-Gruppe können alle zu diesem Zwecke in der Peptid-Chemie gebräuchlichen Gruppen verwendet werden, vgl. das oben zitierte Werk (Houben-Weyl). Bevorzugt sind acidolytisch abspaltbare Gruppen, wie 2-Tetrahydropyranyl und ganz besonders tert.-Butyl. Ferner kann man aber auch reduktiv oder basisch abspaltbare Hydroxyl-Schutzgruppen verwenden, z.B. Benzylgruppen, die im aromatischen Teil durch Halogen, Nitro und/oder Niederalkoxy substituiert sein können, bzw. die Niederalkanoylreste, wie Acetyl oder Aroylreste, wie Benzoyl.

Vorzugsweise werden die Schutzgruppen A und B so gewählt, dass sie unter ähnlichen Bedingungen abspaltbar sind; besonders bevorzugt sind dabei die bereits hervorgehobenen acidolytisch abspaltbaren Gruppen. Die Abspaltung beider Schutzgruppen erfolgt dann vorteilhaft in einer einzigen Operation; es können jedoch auch Gruppen verschiedener Art verwendet werden und jede einzeln abgespalten werden.

Eine durch das Symbol C dargestellte funktionelle Gruppe ergänzt die Carbonylgruppe des C-terminalen Aminosäure-Rests und bildet zusammen mit ihr eine freie Carboxylgruppe, eine aktivierte Estergruppe, beziehungsweise die Carbazolylgruppe.

Die Aktivierungsgruppe, durch welche die Hydroxylgruppe abgewandelt ist, ist insbesondere eine solche, die den aktivierten Ester von N-Hydroxysuccinimid, 1-Hydroxybenzotriazol, N,N'-Dicyclohexylisoharnstoff, 2,4,5-Trichlorphenol, 2-Nitrophenol, 4-Nitrophenol, Pentachlorphenol oder Pentafluorphenol bildet, aber auch eine andere aus der Peptidchemie bekannte Aktivierungsgruppe dieser Art, vgl. Houben-Weyl, Band 15/II.

Unter den linearen Peptiden der Formel III sind diejenigen bevorzugt, worin der Rest Gaba(Ar) als eine endständige Aminosäure im Rest [II'] steht. Diese bevorzugten Ausgangsstoffe sind durch die Formeln

H–Gab(Ar)–Asn–Phe–Phe–trp–Lys(A)–Thr(B)–Phe–C (IIIa)

und insbesondere

H–Asn–Phe–Phe–trp–Lys(A)–Phe–Gaba(Ar)–C (IIIb)

charakterisiert, worin A, B, C, trp und Gaba(Ar) die obengenannten Bedeutungen haben. Ganz besonders bevorzugt sind Verbindungen der Formeln III a und IIIb, worin A für eine Amino-Schutzgruppe, vor allem eine acidolytisch abspaltbare Amino-Schutzgruppe, B für eine Hydroxylschutzgruppe, vor allem eine acidolytisch abspaltbare Hydroxylschutzgruppe und C für eine freie Hydroxylgruppe steht.

Die erfindungsgemässe Cyclisierung der linearen Peptide der Formel III erfolgt in an sich bekannter Weise mittels üblicher, zur Ausbildung der Amid-Bindung gebräuchlicher Kupplungsmethoden, wobei aber die peptidischen Ausgangsstoffe in einer sehr niedrigen Konzentration eingesetzt werden, um den Verlauf der Kupplung zugunsten der intramolekularen Cyclisierung auf Kosten der intermolekularen Polykondensation zu verschieben.

Die linearen Peptide werden mit Vorteil in einer etwa $1.10^{-4}$-molaren bis etwa $1.10^{-2}$-molaren, vorzugsweise einer etwa $1.10^{-3}$-molaren Konzentration eingesetzt, die einer Gewicht/Volumen-Konzentration von etwa 0,01 bis 1,0%, vorzugsweise 0,1% entspricht. Eine entsprechende Verdünnung kann im Reaktionsgemisch vom Anfang an eingestellt werden, oder aber durch langsames Eintropfen des Ausgangsstoffes und gegebenenfalls der übrigen Reagentien in das Reaktionsgemisch fortlaufend hergestellt werden.

Vorzugsweise erfolgt die Cyclisierung, indem man bei einer oben angegebenen Anfangskonzentration a) einen Ausgangsstoff der Formel III, worin C eine freie Hydroxylgruppe bedeutet und in welchem sowohl die ε-Aminogruppe des Lysinrests wie die Hydroxylgruppe des Threoninrests geschützt sind, mit einem Carbodiimid, gegebenenfalls in Anwesenheit einer Aktivester-bildenden Komponente, behandelt, oder b) einen Ausgangsstoff der Formel III, worin C eine zum aktivierten Ester abgewandelte Hydroxylgruppe darstellt und die endständige Aminogruppe in protonierter Form vorliegt, wobei mindestens die ε-Aminogruppe des Lysinrests geschützt ist, mit einer organischen Base umsetzt, oder c) einen Ausgangsstoff der Formel III, worin C die Gruppe $-NHNH_2$ bedeutet und mindestens die ε-Aminogruppe des Lysinrests geschützt ist, zunächst unter sauren Bedingungen mit salpetriger Säure oder einem Niederalkylester davon behandelt und nachher bei einer oben erwähnten niedrigen Konzentration mit überschüssiger organischer Base cyclisiert.

Die Cyclisierung führt man in geeigneten Lösungsmitteln durch; beispielsweise sind Dioxan, Tetrahydrofuran, Acetonitril, Pyridin, Dimethylformamid, Dimethylacetamid, Dimethylsulfoxid, N-Methylpyrrolidon, Hexamethylphosphortriamid, sowie auch Äthylacetat, Chloroform und Methylenchlorid, und Gemische davon zu nennen.

Bei der Verfahrensvariante a) wird die Cyclisierung durch ein Carbodiimid, vorzugsweise N,N'-Dicyclohexylcarbodiimid, das man mit Vorteil im Überschuss anwendet, herbeigeführt; es ist anzunehmen, dass dabei der Ausgangsstoff der Formel III mit freier Carboxylgruppe primär in einen aktivierten Ester des Dicyclohexylisoharnstoffes (bzw. eines analogen Isoharnstoffes) übergeht und dieser in situ gebildete aktive Ester gleich weiterreagiert. Auf eine intermediäre Bildung eines aktiven Esters ist zweifellos die Zugabe einer Aktivester-bildenden Komponente als Hilfsreagens zurückzuführen; zu diesem Zwecke können in der Peptidchemie übliche Aktivester-bildende Komponenten dienen, wie insbesondere 2,4,5-Trichlorphenol, 2- oder 4-Nitrophenol, Pentachlor- und Pentafluorphenol, aber vor allem N-Hydroxyverbindungen, wovon als besonders vorteilhaft N-Hydroxysuccinimid, N-Hydroxypiperidin und vor allem 1-Hydroxybenzotriazol zu erwähnen sind. Die Arbeitstemperatur bei dieser Variante beträgt im allgemeinen 0–70°, vorzugsweise 35–55°.

Bei der Variante b), die mit fertigen Aktivestern, insbesondere den bereits hervorgehobenen, arbeitet, erfolgt die Cyclisierung spontan, sobald die endständige Aminogruppe durch die Base entprotonisiert wird. Die verwendeten Basen sind vorzugsweise quaternäre oder vor allem tertiäre Amine, z.B. Triäthylamin oder N-Äthylmorpholin. Vorzugsweise arbeitet man bei 10–30°, insbesondere bei Raumtemperatur.

Bei der Variante c) kann die erste Phase, d.h. die Bildung des Säureazids durch Behandlung mit salpetriger Säure oder einem Ester davon, mit Vorteil bei einer wesentlich höheren Konzentration der Ausgangsstoffe als die nachfolgende Cyclisierung erfolgen. Zweckmässig arbeitet man mit etwa einem Äquivalent eines Niederalkylnitrits, wie Äthyl-, Isoamyl- und insbesondere tert.-Butyl-nitrit, in salzsaurem Milieu bei Temperaturen von etwa −30° bis etwa −5°, vorzugsweise etwa −20°; ein geringer Überschuss an Nitrit ist zulässig. Danach wird die Lösung des gebildeten Azids nach der notwendigen Verdünnung bei einer Temperatur von etwa 0° bis etwa 35° mittels einer überschüssigen organischen Base, z.B. einer der oben genannten, basisch gestellt und dadurch wie bei der Verfahrensvariante b) zur spontanen Cyclisierung gebracht.

Geht man erfindungsgemäss von einer razemischen γ-Aminobuttersäure H–Gaba(Ar)–OH oder einem Derivat davon aus, so erhält man durch die Kombination mit einer sonst sterisch einheitlichen Teilsequenz 5–11 ein Diastereomerenpaar. Dieses kann durch konventionelle pyhsikalische Trennungsmethoden in individuelle Diastereomere getrennt werden, z.B. durch franktionierte Fällung oder Kristallisation, verschiedene chromatographische Techniken, wie Adsorptions- bzw. Verteilungschromatographie, Gelfiltration und Ionenaustauscher-Trennung, oder vornehmlich durch Verteilung zwischen zwei geeigneten Lösungsmittelsystemen, insbesondere durch die Gegenstromverteilung (Craig-Verfahren). Besonders vorteilhaft verläuft die Trennung nach der letztgenannten Methode bei bereits cyclisierten Verbindungen, d.h. bei den Endstoffen der Formel I und vor allem bei ihren cyclischen Vorgängern der Formel II. (Das dabei isolierte weniger hydrophile Diastereomere wird als Isomer A, das hydrophilere als Isomer B bezeichnet.) Die zu dieser Trennung geeigneten Lösungsmittelsysteme sind in der Peptidchemie allgemein gebräuchlich und bestehen meistens aus einer zweiphasigen Kombination von chlorierten Kohlenwasserstoffen, Niederalkoholen und Wasser, die vornehmlich so gewählt wird, dass beide Phasen in annähernd gleichen Volummengen vorliegen. Diese Systeme stehen in engem Zusammenhang mit denjenigen für Dünnschichtchromatographie und sind von diesen ableitbar (vgl. auch die Durchführungsbeispiele).

Die Abspaltung der Schutzgruppen in II erfolgt in an sich bekannter Weise; die saure Hydrolyse (Acidolyse) wird z.B. mittels Trifluoressigsäure, Salzsäure oder Fluorwasserstoff, bei säureempfindlichen Schutzgruppen auch mittels einer niederaliphatischen Carbonsäure, wie Ameisensäure und/oder Essigsäure, in Anwesenheit von Wasser und gegebenenfalls von einem polyhalogenierten Niederalkanol oder Niederalkanon, wie 1,1,1,3,3,3-Hexafluorpropan-2-ol oder Hexafluoraceton, durchgeführt. Die reduktiv abspaltbaren Gruppen, insbesondere solche, die Benzylreste enthalten, werden vorzugsweise hydrogenolytisch, z.B. durch Hydrierung unter Palladium-Katalyse, entfernt. Die Isonicotinyloxycarbonylgruppe wird vorzugsweise durch Zink-Reduktion abgespalten.

Die erfindungsgemässen Endstoffe werden, je nach Art der Isolierung, als Basen oder als Säureadditionssalze erhalten; diese können nachträglich in an sich bekannter Weise ineinander umgewandelt werden.

Auch die Bildung der oben erwähnten Komplexe erfolgt nach bekannten oder diesen äquivalenten Methoden.

Komplexe mit anorganischen Stoffen wie schwerlöslichen Metall-, z.B. Aluminium- oder Zinkverbindungen werden vorzugsweise in analoger Weise wie für ACTH bekannt, z.B. durch Umsetzung mit einem löslichen Salz des betreffenden Metalls, z.B. Zinkchlorid oder Zinksulfat, und Ausfällung mit einem Alkalimetallphosphat und/oder -hydroxyd hergestellt. Komplexe mit organischen Verbindungen wie Polyoxygelatine, Carboxymethylcellulose, Polyvinylpyrrolidon, Polyphloretinphosphat, Polyglutaminsäure etc. werden durch Mischen dieser Substanzen mit dem Peptid in wässeriger Lösung erhalten. In gleicher Weise können auch unlösliche Verbindungen mit Alkalimetallpolyphosphaten hergestellt werden.

Die Verbindungen der oben charakterisierten Formel II sind neu und gehören auch zum Gegenstand der Erfindung.

Auch die linearen Peptide der Formel III sind neu und gehören auch, sowie ihre Herstellungsverfahren, zum Gegenstand der vorliegenden Erfindung. Sie werden nach an sich bekannten Methoden erhalten, indem man die zu ihrem Aufbau nötigen Aminosäuren bzw. kleinere Peptideinheiten unter Bildung von CONH-Bindungen in beliebiger zeitlicher Reihenfolge miteinander kondensiert, wobei nicht an der Reaktion teilnehmende funktionelle Gruppen intermediär geschützt werden können.

Die oben als bevorzugt hervorgehobenen linearen Peptide der Formeln IIIa und IIIb sind vornehmlich aus Zwischenprodukten zugänglich, deren Herstellung im Zusammenhang mit der Synthese anderer Somatostatin-Analogen in unserer oben erwähnten Europäischen Patentanmeldung, Veröffentlichungsnummer 0 001 295, eingehend beschrieben wurde. Die dort charakterisierten Zwischenprodukte haben bereits die bevorzugte $(D\text{-}trp)^8$-Somatostatin(5–11)-Sequenz in einer geeignet geschützten Form und können mit geeig-

neten Derivaten einer substituierten $\gamma$-Aminobuttersäure der Formel H–Gaba(Ar)–OH direkt kondensiert werden. Die letztgenannten Ausgangsstoffe ihrerseits sind bekannt oder nach bekannten allgemeinen Verfahren zugänglich.

Bei der Herstellung der linearen Peptide der Formel III, wie auch ihrer Zwischenprodukte allgemein, kommen als Schutzgruppen für die endständigen $\alpha$-Amino- und Carboxylgruppen besonders die bei der Synthese langkettiger Peptide üblichen Schutzgruppen in Betracht, die z.B. durch Solvolyse oder Reduktion leicht und selektiv abgespalten werden können.

Als $\alpha$-Amino-Schutzgruppen sind beispielsweise zu nennen: gegebenenfalls, z.B. durch Halogen, Nitro, Niederalkyl oder Niederalkoxy, substituierte Di- oder Triarylniederalkylgruppen, wie Diphenylmethyl- oder Triphenylmethylgruppen, z.B. Benzhydryl, Trityl, Di-(p-methoxy)-benzhydryl, oder vor allem von der Kohlensäure sich ableitende hydrogenolytisch abspaltbare Gruppen, wie gegebenenfalls im aromatischen Rest durch Halogenatome, Nitrogruppen, Niederalkyl- oder Niederalkoxygruppen substituierte Benzyloxycarbonylgruppen, z.B. Benzyloxycarbonyl (d.h. Carbobenzoxy), p-Brom- oder p-Chlorbenzyloxycarbonyl, p-Nitrobenzyloxycarbonyl, p-Methoxybenzyloxycarbonyl; ferner auch 2-(p-Biphenylyl)-2-propyloxycarbonyl und ähnliche im Schweizer Patent 509 266 beschriebene Aryloxycarbonylgruppen. Dabei ist zu beachten, dass die $\alpha$-Aminoschutzgruppe selektiv unter Erhaltung der gegebenenfalls vorhandenen $\varepsilon$-Aminoschutzgruppe des Lysinrests abspaltbar sein muss. Es ist übrigens auch von Vorteil, wenn bei ihrer Abspaltung auch eine gegebenenfalls vorhandene Carboxyl- und Hydroxylschutzgruppe unversehrt bleibt.

Carboxylgruppen werden beispielsweise durch Hydrazidbildung oder durch Veresterung geschützt. Zur Veresterung geeignet sind z.B. niedere, gegebenenfalls substituierte Alkanole wie Methanol, Äthanol, Cyanmethylalkohol, 2,2,2-Trichloräthanol, Benzoylmethylalkohol oder insbesondere tert.-Butylalkohol, aber auch ein gegebenenfalls substituierter Benzylalkohol. Eine besonders vorteilhafte Kategorie der substituierten Alkanole sind Äthylalkohole, die in $\beta$-Stellung eine trisubstituierte Silylgruppe, wie Triphenylsilyl-, Dimethyl-butyl-silyl- oder vor allem Trimethylsilylgruppe, tragen. Wie z.B. im belgischen Patent Nr. 851 576 beschrieben ist, eignen sich diese Alkohole zum Schutze der Carboxylgruppen besonders gut deshalb, weil die entsprechenden $\beta$-Silyläthylester, z.B. $\beta$-(Trimethylsilyl)-äthylester, zwar die Stabilität üblicher Alkylester besitzen, sich jedoch unter milden Bedingungen durch Einwirkung von Fluoridionen unter Erhaltung aller anderen Schutzgruppen selektiv abspalten lassen.

Zur Bildung von aktivierten Estern, wie z.B. in den Verbindungen der Formel III, eignen sich z.B. gegebenenfalls durch elektronenanziehende Substituenten substituierte Phenole und Thiophenole wie Phenol, Thiophenol, Thiokresol, p-Nitrothiophenol, 2,4,5- und 2,4,6-Trichlorphenol, Pentachlorphenol, Pentafluorphenol, o- und p-Nitrophenol, 2,4-Dinitrophenol, p-Cyanophenol, weiter z.B. N-Hydroxysuccinimid, N-Hydroxyphthalimid und N-Hydroxypiperidin.

Die Hydroxylgruppe des Threoninrests kann durch Veresterung oder Verätherung, wie bereits oben angegeben, geschützt werden, sie kann jedoch auch frei bleiben.

Diese Schutzgruppen können in bekannter Weise abgespalten werden. So kann man die Benzyloxycarbonylgruppe durch Hydrogenolyse, die N-Tritylgruppe mit Mineralsäuren, wie Halogenwasserstoffsäuren, z.B. Fluorwasserstoff oder vorzugsweise Chlorwasserstoff, oder einer organischen Säure, wie Ameisensäure, Essigsäure, Chloressigsäure oder Trifluoressigsäure, in wässerigem oder absolutem Trifluoräthanol als Lösungsmittel (vgl. deutsche Offenlegungsschrift DT 2 346 147) oder mit wässeriger Essigsäure, die tert.-Butyloxycarbonylgruppe mit Trifluoressigsäure oder Salzsäure, die 2-(p-Biphenylyl)-isopropyloxycarbonylgruppe mit wässeriger Essigsäure oder z.B. mit einem Gemisch von Eisessig, Ameisensäure (82,8%ig) und Wasser (7 : 1 : 2) oder nach dem Verfahren der DT 2 346 147 abspalten.

Die $\beta$-Silyläthylestergruppen werden vorzugsweise mit Fluoridionen-abgebenden Reagentien, z.B. Fluoriden quaternärer organischer Basen, wie Tetraäthylammoniumfluorid, abgespalten. Sie können aber auch, gleich wie die üblichen Alkylester, durch alkalische Hydrolyse, z.B. mittels Alkalimetallhydroxiden, -carbonaten oder -bicarbonaten, abgespalten werden oder durch Hydrazinolyse, z.B. mittels Hydrazinhydrat, in die entsprechenden Carbazoylgruppen umgewandelt werden. Zur Abspaltung von tert.-Butylestern verwendet man vorzugsweise Acidolyse, für Benzylester dann Hydrogenolyse.

Die zur Herstellung der linearen Peptide der Formel III durchzuführende Kondensation der Aminosäure- und/oder Peptideinheiten erfolgt in an sich bekannter Weise indem man vorzugsweise eine Aminosäure oder ein Peptid mit geschützter $\alpha$-Aminogruppe und gegebenenfalls aktivierter terminaler Carboxylgruppe (= aktive Komponente) an eine Aminosäure oder ein Peptid mit freier $\alpha$-Aminogruppe und freier oder geschützter, z.B. veresterter terminaler Carboxylgruppe (= passive Komponente), anknüpft, im gebildeten Produkt die terminale Aminogruppe freisetzt und dieses Peptid, enthaltend eine freie $\alpha$-Aminogruppe und eine gegebenenfalls geschützte terminale Carboxylgruppe, wieder mit einer weiteren aktiven Komponente, d.h. einer Aminosäure oder einem Peptid mit aktivierter terminaler Carboxylgruppe und freier $\alpha$-Aminogruppe, umsetzt, usw. Die Carboxylgruppe kann beispielsweise durch Überführung in ein Säureazid, -anhydrid, -imidazolid, -isoxazolid oder einen aktivierten Ester, wie einen der oben genannten, oder durch Reaktion mit einem Carbodiimid, wie N,N'-Dicyclohexylcarbodiimid, gegebenenfalls unter Zusatz von N-Hydroxysuccinimid oder einem unsubstituierten oder z.B. durch Halogen, Methyl oder Methoxy substituierten 1-Hydroxybenzotriazol oder 4-Hydroxy-benzo-1,2,3-triazin-3-oxid (u.a., vgl. DT 1 917 690, DT 1 937 656, DT 2 202 613) oder mit N,N'-Carbonyldiimidazol aktiviert werden. Als die gebräuchlichste Kupplungsmethode ist die Carbodiimidmethode zu nennen, ferner auch die Azidmethode, die Methode der aktivierten Ester und die Anhydridmethode, sowie die Merrifield-Methode und die Methode der N-Carboxyanhydride oder N-Thiocarboxyanhydride.

Bei einer besonders bevorzugten Herstellung der linearen Peptide der Formel III verwendet man als die Kupplungsmethode die Carbodiimid-Methode mit N,N'-Dicyclohexylcarbodiimid in Anwesenheit von 1-Hydroxybenzotriazol. Die terminale Carboxylgruppe wird dabei in Form des β-(Trimethyl-silyl)-äthylesters geschützt, die α-Aminogruppe der aktiven Komponente wird durch die Benzyloxycarbonylgruppe geschützt, die nach jedem Kupplungsschritt durch Hydrogenolyse abgespalten wird. Zum Schutz der ε-Aminogruppe des Lysinrests wird die Acylierung mit der tert.-Butoxycarbonylgruppe und für die Hydroxylgruppe des Threoninrests die Verätherung mit der tert.-Butylgruppe verwendet. Diese beiden Schutzgruppen können, wenn erwünscht, zuletzt in einem Schritt durch saure Hydrolyse, z.B. mittels Trifluoressigsäure, Salzsäure oder Fluorwasserstoff, abgespalten werden.

Je nach Arbeitsweise erhält man die Verbindungen in Form von Basen oder ihren Salzen. Aus den Salzen können in an sich bekannter Weise die Basen gewonnen werden. Von letzteren wiederum lassen sich durch Umsetzen mit Säuren, z.B. mit solchen, die die oben genannten Salze bilden, therapeutisch anwendbare Säureadditionssalze gewinnen.

Infolge der engen Beziehungen zwischen den neuen Verbindungen in freier Form und in Form ihrer Salze sind im vorausgegangenen und nachfolgend unter den freien Verbindungen oder ihren Salzen sinn- und zweckgemäss gegebenenfalls auch die entsprechenden Salze bzw. freien Verbindungen zu verstehen.

Die Erfindung betrifft auch diejenigen Ausführungsformen des Verfahrens, bei denen man von einer auf beliebiger Verfahrensstufe als Zwischenprodukt erhältlichen Verbindung ausgeht und die fehlenden Verfahrensschritte ausführt, oder wobei ein Ausgangsstoff unter den Reaktionsbedingungen gebildet oder in Form eines Derivats davon, gegebenenfalls eines Salzes, verwendet wird.

Beim Verfahren der vorliegenden Erfindung werden vorzugsweise solche Ausgangsstoffe verwendet, welche zu den eingangs als besonders wertvoll geschilderten Verbindungen führen.

Die vorliegende Erfindung betrifft ebenfalls pharmazeutische Präparate, welche Verbindungen der Formel I oder pharmazeutisch verwendbare Salze davon enthalten. Diese pharmazeutischen Präparate können insbesondere in den oben angegebenen Indikationen Verwendung finden, wenn sie intraperitoneal, wie intravenös, intramuskulär oder subcutan, oder auch intranasal verabreicht werden. Die erforderliche Dosis hängt von der speziellen zu behandelnden Erkrankung, ihrer Schwere und von der Dauer der Therapie ab. Anzahl und Menge der Einzeldosen, sowie das Verabreichungsschema kann am besten anhand einer individuellen Untersuchung des jeweiligen Patienten bestimmt werden. Die Methode zur Bestimmung dieser Faktoren ist dem Fachmann geläufig. In der Regel liegt jedoch bei einer Injektion eine therapeutisch wirksame Menge einer solchen Verbindung im Dosisbereich von etwa 0,001 bis etwa 0,2 mg/kg Körpergewicht vor. Bevorzugt wird der Bereich von etwa 0,0015 bis etwa 0,15 mg/kg Körpergewicht und die Verabreichung erfolgt durch intravenöse Infusion oder subkutane Injektion. Dementsprechend enthalten pharmazeutische Präparate zur parenteralen Verabreichung in Einzeldosis-Form in Abhängigkeit von der Applikationsart pro Dosis etwa 0,08 bis etwa 15 mg einer der erfindungsgemässen Verbindungen. Neben dem Wirkstoff enthalten sie üblicherweise noch einen Puffer, z.B. einen Phosphatpuffer, der das pH zwischen etwa 3,5 und 7 halten soll, ferner Natriumchlorid, Mannit oder Sorbit zur Einstellung der Isotonie. Sie können in gefriergetrockneter oder gelöster Form vorliegen, wobei Lösungen mit Vorteil ein antibakteriell wirkendes Konservierungsmittel, z.B. 0,2–0,3% 4-Hydroxybenzoesäuremethylester oder -äthylester enthalten können. Soll in solchen Präparaten der Wirkstoff in Form eines Komplexes mit verlängerter Wirkungsdauer vorliegen, so kann dieser direkt durch Zusatz der komplexbildenden Komponenten zu einer Injektionslösung, die z.B. gemäss den oben erwähnten Massnahmen zubereitet wird, gebildet werden. Als Zusatz eignet sich z.B. 0,1–1,0 Gewichts-% eines Zink(II)-Salzes (z.B. Sulfats) in Verbindung mit 0,5–5,0 Gew.-% Protamin (z.B. als Sulfat), bezogen auf das Gesamtvolumen der Injektionslösung; diese Zubereitung liegt als Lösung von pH 3,5 bis etwa 6,5 oder als Suspension von etwa pH 7,5 bis 8,0 vor.

Ein Präparat für die intranasale Verabreichung kann als eine wässrige Lösung oder Gelee, eine ölige Lösung oder Suspension, oder aber eine fetthaltige Salbe vorliegen. Ein Präparat in Form einer wässrigen Lösung erhält man z.B. dadurch, dass man den Wirkstoff der Formel I, oder ein therapeutisch anwendbares Säureadditionssalz davon in einer wässrigen Pufferlösung von pH bis 7,2 löst und einen Isotonie-erzeugenden Stoff zusetzt. Der wässrigen Lösung wird zweckmässig ein polymeres Haftmittel, z.B. Polyvinylpyrrolidon, und/oder ein Konservierungsmittel zugefügt. Die Einzeldosis beträgt etwa 0,08 bis etwa 15 mg, vorzugsweise 0,25 bis 10 mg, die in etwa 0,05 ml einer Lösung bzw. 0,05 g eines Gelees enthalten sind.

Eine ölige Applikationsform für die intranasale Verabreichung erhält man z.B. durch Suspendieren eines Peptides der Formel I oder eines therapeutisch anwendbaren Säureadditionssalzes davon in einem Öl, gegebenenfalls unter Zusatz von Quellmitteln, wie Aluminiumstearat, und/oder grenzflächenaktiven Mitteln (Tensiden), deren HLB-Wert («hydrophilic-lipophilic-balance») unter 10 liegt, wie Fettsäuremonoester mehrwertiger Alkohole, z.B. Glycerinmonostearat, Sorbitanmonolaurat, Sobritanmonostearat oder Sorbitanmonoleat. – Eine fetthaltige Salbe erhält man z.B. durch Suspendieren des erfindungsgemässen Wirkstoffes in einer streichbaren Fettgrundlage, gegebenenfalls unter Zusatz eines Tensids vom HLB-Wert unter 10. Eine Emulsionssalbe erhält man durch Verreiben einer wässrigen Lösung des peptidischen Wirkstoffes in einer weichen, streichbaren Fettunterlage unter Zusatz eines Tensids, dessen HLB-Wert unter 10 liegt. Alle diese intranasale Applikationsformen können auch Konservierungsmittel enthalten. – Die Einzeldosen betragen etwa 0,08 bis etwa 15 mg, vorzugsweise 0,25 bis 10 mg, enthalten in etwa 0,05 bis etwa 0,1 g der Grundmasse.

Für die intranasale Verabreichung eignen sich weiter auch Inhalations- bzw. Insufflationspräparate, wie Insufflationskapseln, die den Wirkstoff in Form eines Puders mit der Atemluft zu insufflieren gestatten, oder Aerosole oder Sprays, welche den pharmakologischen Wirkstoff in Form eines Puders oder in Form von Tropfen einer Lösung oder Suspension verteilen können. Präparate mit Puder-verteilenden Eigenschaften enthalten ausser dem Wirkstoff üblicherweise Hilfsmittel: Insufflationskapseln enthalten z.B. feste Trägerstoffe, wie Lactose; Aerosol- bzw. Sprayzubereitungen enthalten z.B. ein flüssiges Treibgas mit einem Siedepunkt unter der Raumtemperatur, sowie, wenn erwünscht, weitere Trägerstoffe, wie flüssige oder feste nichtionische oder anionische Tenside und/oder feste Verdünnungsmittel. Präparate, in welchen der pharmakologische Wirkstoff in Lösung vorliegt, enthalten ausser diesem ein geeignetes Treibmittel, ferner, falls notwendig, ein zusätzliches Lösungsmittel und/oder einen Stabilisator. Anstelle des Treibgases kann auch Druckluft verwendet werden, die mittels einer geeigneten Verdichtungs- und Entspannungsvorrichtung nach Bedarf erzeugt wird.

Die Erfindung betrifft ebenfalls die Verwendung der neuen Verbindungen der Formel I und therapeutisch anwendbarer Säureadditionssalzen davon als pharmakologisch aktive Verbindungen, insbesondere in den für Somatostatin üblichen Indikationen, vorzugsweise in der Form von pharmazeutischen Präparaten. Die tägliche Dosis, die einem Warmblüter von etwa 70 kg verabreicht wird, beträgt von etwa 0,1 bis etwa 120 mg.

In den nachfolgenden Beispielen wird die Erfindung illustriert, jedoch durch diese nicht eingeschränkt. Temperaturen werden in Celsiusgraden angegeben; als Abkürzungen, z.B. zur Bezeichnung von Aminosäuren, Peptiden, Schutzgruppen, etc., werden die üblichen, z.B. die in «Synthese von Peptiden» (Herausgeber: E. Wünsch), Bd XV der «Methoden der org. Chemie» (Houben-Weyl)(1974; G. Thieme, Stuttgart), zusammengestellten Kurzbezeichnungen verwendet. Insbesondere werden folgende Abkürzungen verwendet.

| | |
|---|---|
| Boc | tert.-Butoxycarbonyl |
| But | tert.-Butyl (als ätherbildende Gruppe) |
| DCC | N,N'-Dicyclohexylharnstoff |
| DCCI | N,N'-Dicyclohexylcarbodiimid |
| Gaba | 4-Aminobuttersäure-Rest, $-NH-(CH_2)_3-CO-$ |
| OBzl | Benzyloxy (als esterbildende Gruppe) |
| Z | Benzyloxycarbonyl (Carbobenzoxy) |
| CD | Circulardichroismus |
| ORD | optische Rotationsdispersion |
| DC | Dünnschichtchromatographie |

In DC verwendet man, wenn nichts anderes angegeben ist, Kieselgel als Adsorbens und folgende Systeme als Laufmittel.

System

| | |
|---|---|
| 52: | n-Butanol-Essigsäure-Wasser (75 : 7, 5 : 21) |
| 101: | n-Butanol-Pyridin-Essigsäure-Wasser (38 : 24 : 8 : 30) |
| 111B: | n-Butanol-Pyridin-25%iges wässeriges Ammoniak-Wasser (40 : 24 : 6 . 30) |
| 112A: | n-Butanol-Pyridin-Ameisensäure-Wasser (42 : 24 : 4 : 20). |
| 157A: | Chloroform-Methanol-Wasser-Essigsäure (90 : 10 : 1 : 0,5) |
| 157C: | Chloroform-Methanol-Wasser-Essigsäure (75 : 27 : 5 : 0,5) |
| 157F: | Chloroform-Methanol-Wasser-Essigsäure (70 : 40 : 9 : 0,5). |

Beispiel 1A

–Asn–Phe–Phe–(D–trp)–Lys–Thr–Phe–(β-Phenyl)Gaba– –Isomer A

160 mg geschütztes Octapeptid der Formel

–Asn–Phe–Phe–(D–trp)–Lys(Boc)–Thr(But)–Phe–(β-Phenyl)Gaba– –Isomer A

wird bei 5° unter $N_2$ in 1,5 ml eines Gemisches aus 89 Vol.-% Trifluoressigsäure, 10 Vol.-% Wasser und 1 Vol.-% Thioglykolsäure gelöst, die Lösung sofort auf 25° erwärmt und nach 50 Minuten bei dieser Temperatur mit 5 ml peroxidfreiem Äther ausgefällt. Das resultierende rohe Trifluoracetat des Endprodukts wird abfiltriert, im Vakuum getrocknet, in 5 ml 1N Essigsäure gelöst und durch 15 ml Anionenaustauscher, z.B. AG® 1–X8 (ein Produkt von Bio-Rad Laboratories, Richmond, Calif., USA), in Acetaform, filtriert; das Eluat wird lyophilisiert.

Die erhaltene Titelverbindung ist in drei Systemen dünnschichtchromatographisch einheitlich.

DC (Cellulose, Merck): System 101 : $R_f$ 0,66
111B : $R_f$ 0,48
112A : $R_f$ 0,60

CD (in Wasser): λ(nm)/mol. Ellipt.: 235/−5800 (min); 223/+6600 (max).

In analoger Weise werden auch die folgenden Endstoffe der Formel

–Asn–Phe–Phe–(D–trp)–Lys–Thr–Phe–(Ar)Gaba–

aus entsprechenden geschützten Octapeptiden erhalten:

(Ar)Gaba = β-(2-Naphthyl)Gaba – Isomer A
DC: System 157C: $R_f$ 0,27;
(Ar)Gaba = β-(2-Naphthyl)Gaba – Isomer B
DC: System 157C: $R_f$ 0,27;
(Ar)Gaba = β-(4-Fluorphenyl)Gaba – Isomer B
DC: System 157C: $R_f$ 0,29.

Beispiel 1B

└Asn–Phe–Phe–(D–trp)–Lys–Thr–Phe–
(β-Phenyl)Gaba┘ -Isomer B

189 mg geschütztes Octapeptid der Formel

└Asn–Phe–Phe–(D–trp)–Lys(Boc)–Thr(But)–
Phe–(β-Phenyl)Gaba┘ -Isomer B

wird bei 5° unter $N_2$ in 1,9 ml eines Gemisches aus 89 Vol.-% Trifluoressigsäure, 10 Vol.-% Wasser und 1 Vol.-% Thioglykolsäure gelöst, die Lösung sofort auf 25° erwärmt und nach 50 Minuten bei dieser Temperatur mit 5 ml peroxidfreiem Äther ausgefällt. Das resultierende rohe Trifluoracetat des Endprodukts wird abfiltriert, im Vakuum getrocknet, in 5 ml 1 N Essigsäure gelöst und durch 15 ml Anionenaustauscher, z.B. AG® 1–X8 (ein Produkt von Bio-Rad Laboratories, Richmond, Calif., USA), in Acetatform, filtriert. Das Eluat wird im Vakuum eingedampft und der Rückstand der Gegenstromverteilung im System n-Butanol-Essigsäure-Wasser (2400 : 600 : 3000) über 270 Stufen unterzogen. Die in den Elementen 229 bis 250 enthaltenen Phasen (K = 6,7) werden gesammelt, im Vakuum eingedampft und aus tert.-Butanol-Wasser (1 . 1) lyophilisiert.

Die erhaltene Titelverbindung ist in drei Systemen dünnschichtchromatographisch einheitlich.

DC (Cellulose, Merck) System 101 : $R_f$ 0,64
111B : $R_f$ 0,48
112A : $R_f$ 0,58

CD: (in Wasser): λ(nm)/mol. Ellipt.: 235/–3700 (min); 220/ – 35000 (max).

Die peptidischen Ausgangsstoffe der Beispiele 1A und 1B sind folgendermassen erhältlich:

Stufe 1.1
d,l-H-(β-Phenyl)Gaba-OBzl-p-toluolsulfonat

Ein Gemisch von 1,10 g d,l-4-Amino-3-phenylbuttersäure und 1,17 g p-Toluolsulfonsäure-monohydrat in 3,2 ml Benzylalkohol und 50 ml Benzol wird bei Normaldruck langsam destilliert, bis während 3 Stunden insgesamt 40 ml einer Fraktion, Sdp. 70°–90° aufgefangen wird. Die klare Reaktionslösung wird im Wasserstrahlvakuum und dann im Hochvakuum bei ca. 60° auf 3 ml konzentriert. Der ausgefallene Kristallbrei wird mit 15 ml Äther verrührt, die Kristalle abfiltriert und mit 10 ml Äther gewaschen. Zur weiteren Reinigung wird dieses Material mit 10 ml Äther bei Raumtemperatur während einer Stunde verrührt, abfiltriert, mit Äther gewaschen und im Vakuum getrocknet, Smp. 145–8°.

DC: [Chloroform-Methanol-Wasser (14 . 6 : 1)] $R_f$ 0,40
[Chloroform-Methanol (85 : 15)] $R_f$ 0,17

In analoger Weise wird auch erhalten.

a) aus d,l-4-Amino-3-(2-naphthyl)-buttersäure des d,l-H-[β-(2-Naphthyl)] Gaba-OBzl-p-toluolsulfonat,
DC: System 157C: $R_f$ 0,48; und

b) aus d,l-4-Amino-3-(4-fluorphenyl)-buttersäure das d,l-H-[β-(4-Fluorphenyl)]Gaba-OBzl-p-toluolsulfonat,
DC: System 157: $R_f$ 0,45.

Stufe 1.2
Z–Asn–Phe–Phe–(D–trp)–Lys(Boc)–Thr(But)–Phe–[d,l-β-Phenyl)Gaba]-OBsl

Ein Gemisch von 500 mg Z–Asn–Phe–Phe–(D–trp)–Lys(Boc)–Thr(But)–Phe–OH (siehe die oben zitierte Europäische Patentanmeldung Beispiel 1, Stufe 1.7) und 189 mg d,l-4-Amino-3-phenylbuttersäure-benzylester-p-toluolsulfonat (Stufe 1.1) in 2 ml Dimethylformamid wird mit 0,048 ml N-Methylmorpholin, 58 mg N-Hydroxybenzotriazol und 104 ml DCCI versetzt und 20 Stunden bei Raumtemperatur belassen. Zur Aufarbeitung wird vom ausgefallenen DCC abzentrifugiert, der Überstand mit 15 ml Wasser versetzt und abfiltriert. Der gewonnene Feststoff wird zur weiteren Reinigung mit 3 ml Äthanol während 10 Minuten verrührt, die Suspension auf 0° abgekühlt, das reine Produkt abfiltriert und im Vakuum getrocknet.

DC: [Chloroform-Methanol (85 : 15)] $R_f$ 0,70.

In analoger Weise werden aus entsprechenden Verbindungen der Stufe 1.1 auch erhalten:

a) Z–Asn–Phe–Phe–(D–trp)–Lys(Boc)–Thr(But)–Phe–[d,l-{β-(2-Naphthyl)]Gaba}-OBzl,
DC: System 157A: $R_f$ 0,65; und

b) Z–Asn–Phe–Phe–(D–trp)–Lys(Boc)–Thr(But)–Phe–{d,l-(β-(4-Fluorphenyl)]Gaba}-OBzl,
DC: System 157A: $R_f$ 0,64.

Stufe 1.3
H–Asn–Phe–Phe–(D–trp)–Lys(Boc)–Thr(But)–Phe–[d,l-(β-Phenyl)Gaba]-OH

Eine Lösung von 453 mg Z–Asn–Phe–Phe–(D–trp)–Lys(Boc)–Thr(But)–Phe–[d,l-(β-Phenyl)Gaba]-OBzl (Stufe 1.2) in 20 ml Dimethylformamid wird nach Zugabe von 50 mg Palladium-Kohle (10%) während 3 Stunden bei Raumtemperatur und Normaldruck hydriert. Zur Aufarbeitung wird die Lösung nach Abfiltrieren des Katalysators im Hochvakuum auf 2 ml konzentriert und das Produkt mit 25 ml peroxidfreiem Äther ausgefällt, abfiltriert und im Vakuum getrocknet. Das Rohprodukt wird ohne weitere Reinigung der nächsten Stufe 1.4 (Cyclisierung) unterzogen.

In analoger Weise werden aus entsprechenden Verbindungen der Stufe 1.2 erhalten:

a) H–Asn–Phe–Phe–(D–trp)–Lys(Boc)–Thr(But)–Phe–{d,l-[β-(2-Naphthyl)]Gaba}-OH;
DC: System 157C: $R_f$ 0,67; und

b) H–Asn–Phe–Phe–(D–trp)–Lys(Boc)–Thr(But)–Phe–{d,l-[β-(4-Fluorphenyl)]Gaba}-OH,
DC: System 157A: $R_f$ 0,06.

Stufe 1.4

Asn–Phe–Phe–(D–trp)–Lys(Boc)–Thr(But)–Phe–(β-Phenyl)Gaba

Eine Lösung von 386 mg rohem H–Asn–Phe–Phe–(D–trp)–Lys(Boc)–Thr(But)–Phe–[d,l-(β-Phenyl)Gaba]-OH (Stufe 1.3), 400 mg N-Hydroxybenzotriazol und 610 mg DCCI in 300 ml Dimethylformamid wird während 20 Stunden bei 50 °C gehalten. Zur Aufarbeitung wird das Lösungsmittel im Hochvakuum bei ca. 30° abgedampft und der Rückstand mit 15 ml Äthylacetat verrieben. Der ausgefallene Dicyclohexylharnstoff wird durch Abfiltrieren beseitigt, das Filtrat mit Äthylacetat auf 50 ml verdünnt, dreimal mit je 40 ml 1N wässeriger Oxalsäure und dann mit Wasser bis zur Neutralität gewaschen, über Natriumsulfat getrocknet und im Vakuum eingedampft. Das Rohprodukt wird zur Reinigung und Trennung der Diastereomeren A und B der Gegenstromverteilung im System Methanol-Wasser-Chloroform-Tetrachlorkohlenstoff (2700 : 675 . 900 : 1575 Vol.-Teile) über 440 Stufen unterzogen. Die in den Elementen 104 bis 155 befindlichen Phasen (K = 0.41) enthalten eines der beiden Diastereomeren (Isomer A), die in den Elementen 164–207 (K = 0.70) befindlichen Phasen dann das andere (Isomer B). Die Phasen der entsprechenden Elemente werden vereinigt und im Vakuum eingedampft. Der Rückstand wird in 20 ml tert.-Butanol gelöst und lyophilisiert, womit jeweils ein dünnschichtchromatographisch einheitliches Diastereomeres der obigen gemeinsamen Formel resultiert.

DC: [Chloroform-Methanol (85 . 15)] Isomer A: $R_f$ 0,65; Isomer B: $R_f$ 0,55
[Chloroform-Methanol-Wasser (14 : 6 : 1)] Isomer A: $R_f$ 0,95: Isomer B: $R_f$ 0,90.

In analoger Weise werden aus entsprechenden Verbindungen der Stufe 1.3 enthalten:

a) Asn–Phe–Phe–(D–trp)–Lys(Boc)–Thr-(But)–Phe–(β-(2-Naphthyl)]Gaba,

Isomer A: K = 0,27; DC: System 157A: $R_f$ 0,39
Isomer B: K = 0,51; DC. System 157A: $R_f$ 0,32, sowie

b) Asn–Phe–Phe–(D–trp)–Lys(Boc)–Thr(But)–Phe–[β-(4-Fluorphenyl)]Gaba,

Isomer B: K = 0,78; DC: System 157A: $R_f$ 0,31.

Beispiel 2A

Asn–Phe–Phe–(D–trp)–Lys–Thr–Phe–[β-(1-Naphthyl)]Gaba -Isomer A.

250 mg geschütztes Octapeptid der Formel

Asn–Phe–Phe–(D–trp)–Lys(Boc)–Thr(But)–Phe–[β(1-Naphthyl)]Gaba -Isomer A

wird bei 5° unter $N_2$ in 2,5 ml eines Gemisches aus 89 Vol.-% Trifluoressigsäure, 10 Vol.-% Wasser und 1 Vol.-% Thioglykolsäure gelöst, die Lösung sofort auf 25° erwärmt und nach 50 Minuten bei dieser Temperatur mit 8 ml peroxidfreiem Äther ausgefällt. Das resultierende rohe Trifluoracetat des Endprodukts wird abfiltriert, im Vakuum getrocknet, in 5 ml 1 N Essigsäure gelöst und durch 15 ml Anionenaustauscher, z.B. AG® 1–X8 (ein Produkt von Bio-Rad Laboratories, Richmond, Calif., USA), in Acetatform, filtriert; das Eluat wird lyophilisiert.

Die erhaltene Titelverbindung ist in drei Systemen dünnschichtchromatographisch einheitlich.

DC (Cellulose, Merck): System 101 : $R_f$ 0,60
111B : $R_f$ 0,45
112A : $R_f$ 0,58

CD (in 1%-wässriger Essigsäure): λ(nm)/mol. Ellipt.: 226/ − 8000 (min).

Beispiel 2B

Asn–Phe–Phe–(D–trp)–Lys–Thr–Phe–[β-(1-Naphthyl)]Gaba -Isomer B

257 mg geschütztes Octapeptid der Formel

Asn–Phe–Phe–(D–trp)–Lys(Boc)–Thr(But)–Phe–[β-(1-Naphthyl)]Gaba -Isomer B

wird bei 5° unter $N_2$ in 2,5 ml eines Gemisches aus 89 Vol.-% Trifluoressigsäure, 10 Vol.-% Wasser und 1 Vol.-% Thioglykolsäure gelöst, die Lösung sofort auf 25° erwärmt und nach 50 Minuten bei dieser Temperatur mit 10 ml peroxidfreiem Äther ausgefällt. Das resultierende rohe Trifluoracetat des Endprodukts wird abfiltriert, im Vakuum getrocknet, in 5 ml 1 N Essigsäure gelöst und durch 15 ml Anionenaustauscher, z.B. AG® 1–X8 (ein Produkt von Bio-Rad Laboratories, Richmond, Calif., USA), in Acetatform, filtriert; das Eluat wird lyophilisiert.

Die erhaltene Titelverbindung ist in drei Systemen dünnschichtchromatographisch einheitlich.

DC (Cellulose, Merck): System 101 : $R_f$ 0,57
111B : $R_f$ 0,44
112A : $R_f$ 0,53

CD (in 1%-wässriger Essigsäure): λ(nm)/mol.-Ellipt.: 237/ + 6500 (max); 231/–7000 (min); 223/ + 12000 (max).

Der peptidische Ausgangsstoff der Beispiele 2A und 2B ist folgendermassen erhältlich:

Stufe 2.1
d,l-H-(β-(1-Naphthyl)]Gaba-OBzl-p-toluolsulfonat

Ein Gemisch von 1,05 g d,l-4-amino-3-(1-naphthyl)-buttersäure und 0,871 g p-Toluolsulfonsäure-monohydrat in 2,4 ml Benzylalkohol und 50 ml Benzol wird bei Normaldruck langsam destilliert, bis während 3 Stunden insgesamt 40 ml einer Fraktion, Sdp. 70–90° aufgefangen wird. Die klare Reaktionslösung wird im Wasserstrahlvakuum und dann im Hochvakuum bei ca. 60° auf 3 ml konzentriert. Der ausgefallene Kristallbrei wird mit 15 ml Äther verrührt, die Kristalle abfiltriert und mit 10 ml Äther gewaschen. Zur weiteren Reinigung wird dieses Material mit 10 ml Äther bei Raumtemperatur während einer Stunde verrührt, abfiltriert, mit Äther gewaschen und im Vakuum getrocknet, Smp. 152–4°.

DC: [Chloroform-Methanol-Wasser (14 : 6 : 1) $R_f$ 0,60
[Chloroform-Methanol (85 . 15)] $R_f$ 0,28

Stufe 2.2
Z–Asn–Phe–Phe–(D–trp)–Lys(Boc)–Thr(But)–Phe–[d,l-β-(1-Naphthyl)]Gaba-OBzl

Ein Gemisch von 700 mg
Z–Asn–Phe–Phe–(D–trp)–Lys(Boc)–Thr(But)–Phe–OH
(vgl. Stufe 1.2) und 295 mg
d,l-4-Amino-3-(1-naphthyl)-buttersäure-benzylester-p-toluolsulfonat
(Stufe 2.1) in 2 ml Dimethylformamid wird mit 0,067 ml N-Methylmorpholin, 81 mg N-Hydroxybenzotriazol und 147 mg DCCl versetzt und 20 Stunden bei Raumremperatur belassen. Zur Aufarbeitung wird vom ausgefallenen DCC abzentrifugiert, der Überstand mit 20 ml Wasser versetzt und abfiltriert. Der gewonnene Feststoff wird zur weiteren Reinigung mit 3 ml Methanol während 10 Minuten verrührt, die Suspension auf 0° abgekühlt, das reine Produkt abfiltriert und im Vakuum getrocknet.

DC: [Chloroform-Methanol (85 : 15)] $R_f$ 0,72
[Chloroform-Methanol-Wasser (14 : 6 : 1) $R_f$ 0,93

Stufe 2.3
H–Asn–Phe–Phe–(D–trp)–Lys(Boc)–Thr(But)–Phe–[d,l-β-(1-Naphthyl)]Gaba-OH

Eine Lösung von 794 mg
Z–Asn–Phe–Phe–(D–trp)–Lys(Boc)–Thr(But)–Phe–[d,l–β–(1-Naphthyl)]Gaba–OBzl
(Stufe 2.2) in 40 ml Dimethylformamid wird nach Zugabe von 80 mg Palladium-Kohle (10%) während 4 Stunden bei Raumtemperatur und Normaldruck hydriert. Zur Aufarbeitung wird die Lösung nach Abfiltrieren des Katalysators im Hochvakuum auf 2 ml konzentriert und das Produkt mit 40 ml peroxidfreiem Äther ausgefällt, abfiltriert und im Vakuum getrocknet. Das Rohprodukt wird ohne weitere Reinigung der nächsten Stufe 2.4 (Cyclisierung) unterzogen.

Stufe 2.4

└Asn–Phe–Phe–(D–trp)–Lys(Boc)–Thr(But)–Phe–[β-(1-Naphthyl)]Gaba┘

Eine Lösung von 678 mg rohem
H–Asn–Phe–Phe–(D–trp)–Lys(Boc)–Thr(But)–Phe–[d,l–β-(1-Naphthyl)]Gaba-OH
(Stufe 2.3), 676 mg N-Hydroxybenzotriazol und 1030 mg DCCl in 500 ml Dimethylformamid wird während 20 Stunden bei 50° gehalten. Zur Aufarbeitung wird das Lösungsmittel im Hochvakuum bei ca. 30° abgedampft und der Rückstand mit 20 ml Äthylacetat verrieben. Der ausgefallene Dicyclohexylharnstoff wird durch Abfiltrieren beseitigt, das Filtrat mit Äthylacetat auf 200 ml verdünnt, dreimal mit je 50 ml 1N wässeriger Oxalsäure und dann mit Wasser bis zur Neutralität gewaschen, über Natriumsulfat getrocknet und im Vakuum eingedampft. Das Rohprodukt wird zur Reinigung der Gegenstromverteilung im System Methanol-Wasser-Chloroform-Tetrachlorkohlenstoff (2700 : 675 : 900 : 1575 Vol.-Teile) über 560 Stufen unterzogen. Die in den Elementen 89 bis 143 befindlichen Phasen (K = 0,22) enthalten eines der beiden Diastereomeren (Isomer A), die in den Elementen 152–224 befindlichen Phasen (K = 0,47) dann das andere (Isomer B). Die Phasen der entsprechenden Elemente werden vereinigt und im Vakuum eingedampft. Der Rückstand wird im 20 ml tert.-Butanol gelöst und lyophilisiert, womit jeweils ein dünnschichtchromatographisch einheitliches Diastereomeres der obigen gemeinsamen Formel resultiert.

DC: [Chloroform-Methanol (85 :15)] Isomer A: $R_f$ 0,60; Isomer B: $R_f$ 0,50.

Beispiel 3A

└Asn–Phe–Phe–(D–trp)–Lys–Thr–Phe–(β-Cyclohexyl)Gaba┘-Isomer A.

182 mg geschütztes Octapeptid der Formel

└Asn–Phe–Phe–(D–trp)–Lys(Boc)–Thr(But)–Phe–(β-Cyclohexyl)Gaba┘-Isomer A

wird bei 5° unter $N_2$ in 1,8 ml eines Gemisches aus 89 Vol.-% Trifluoressigsäure, 10 Vol.-% Wasser und 1 Vol.-% Thioglykolsäure gelöst, die Lösung sofort auf 25° erwärmt und nach 50 Minuten bei dieser Temperatur mit 6 ml Hexan-Äther (2 : 1) ausgefällt. Das resultierende rohe Trifluoracetat des Endprodukts wird abfiltriert, im Vakuum getrocknet, in 5 ml 1 N Essigsäure gelöst und durch 15 ml Anionenaustauscher, z.B. AG® 1-X8 (ein Produkt von Bio-Rad Laboratories, Richmond, Calif., USA), in Acetatform, filtriert; das Eluat wird lyophilisiert.

Die erhaltene Titelverbindung ist in drei Systemen dünnschichtchromatographisch einheitlich.

DC: System 101 : $R_f$ 0,88
111B : $R_f$ 0,50
112A : $R_f$ 0,78.

Beispiel 3B

Asn–Phe–Phe–(D–trp)–Lys–Thr–Phe–(β-Cyclohexyl)Gaba -Isomer B

167 mg geschütztes Octapeptid der Formel

Asn–Phe–Phe–(D–trp)–Lys(Boc)–Thr(But)–Phe–(β-Cyclohexyl)Gaba -Isomer B

wird bei 5° unter $N_2$ in 1,7 ml eines Gemisches aus 89 Vol.-% Trifluoressigsäure, 10 Vol.-% Wasser und 1 Vol.-% Thioglykolsäure gelöst, die Lösung sofort auf 25° erwärmt und nach 50 Minuten bei dieser Temperatur mit 5 ml Hexan-Äther (2 : 1 ausgefällt. Das resultierende rohe Trifluoracetat des Endprodukts wird abfiltriert, im Vakuum getrocknet, in 5 ml 1 N Essigsäure gelöst und durch 15 ml Anionenaustauscher, z.B. AG® 1–X8 (ein Produkt von Bio-Rad Laboratories, Richmond, Calif., USA), in Acetatform, filtriert; das Eluat wird lyophilisiert.

Die erhaltene Titelverbindung ist in drei Systemen dünnschichtchromatographisch einheitlich.

DC: System 101 : $R_f$ 0,83
111B : $R_f$ 0,75
112A : $R_f$ 0,70.

Der peptidische Ausgangsstoff der Beispiele 3A und 3B ist folgendermassen erhältlich:

Stufe 3.1

d,l-H-(β-Cyclohexyl)Gaba-OBzl-p-toluolsulfonat

Ein Gemisch von 0,50 g d,l-4-Amino-3-cyclohexyl-buttersäure-hydrochlorid und 0,428 g p-Toluolsulfonsäure-monohydrat in 1,2 ml Benzylalkohol und 40 ml Benzol wird bei Normaldruck langsam destilliert, bis während 3 Stunden insgesamt 30 ml einer Fraktion, Sdp. 70–90°, aufgefangen wird. Die klare Reaktionslösung wird im Wasserstrahlvakuum und dann im Hochvakuum bei ca. 60° auf 3 ml konzentriert. Der ausgefallene Kristallbrei wird mit 15 ml Äther verrührt, die Kristalle abfiltriert und mit 10 ml Äther gewaschen. Zur weiteren Reinigung wird dieses Material mit 10 ml Äther bei Raumtemperatur während einer Stunde verrührt, abfiltriert, mit Äther gewaschen und im Vakuum getrocknet.

DC: [Chloroform-Methanol-Wasser (14 : 6 : 1)]$R_f$ 0,48
[Chloroform-Methanol (85 : 15)] $R_f$ 0,15.

Stufe 3.2

Z–Asn–Phe–Phe–(D–trp)–Lys(Boc)–Thr(But)–Phe–(d,l-β-Cyclohexyl)Gaba-OBzl

Ein Gemisch von 1,06 g
Z–Asn–Phe–Phe–(D–trp)–Lys(BocZ–Thr(But)–Phe–OH
(vgl. Stufe 1.2) und 448 mg
d,l-4-Amino-3-cyclohexyl-buttersäure-benzylester-p-toluolsulfonat
(Stufe 3.1) in 5 ml Dimethylformamid wird mit 0,111 ml N-Methylmorpholin, 123 mg N-Hydroxybenzotriazol und 223 mg DCCI versetzt und 20 Stunden bei Raumtemperatur belassen. Zur Aufarbeitung wird vom ausgefallenen DCC abzentrifugiert, der Überstand mit 20 ml Wasser versetzt und abfiltriert. Der gewonnene Feststoff wird zur weiteren Reinigung mit 3 ml Methanol während 10 Minuten verrührt, die Suspension auf 0° abgekühlt, das reine Produkt abfiltriert und im Vakuum getrocknet.

DC: [Chloroform-Methanol (85 : 15)] $R_f$ 0,80
[Chloroform-Methanol-Wasser (14 : 6 : 1)] $R_f$ 0,95.

Stufe 3.3

H–Asn–Phe–Phe–(D–trp)–Lys(Boc)–Thr(But)–Phe–(d,l-β-Cyclohexyl)Gaba-OH

Eine Lösung von 980 mg
Z–Asn–Phe–Phe–(D trp)–Lys(Boc)–Thr(But)–Phe–(d,l-β-Cyclohexyl)Gaba-OBzl
(Stufe 3.2) in 50 ml Dimethylformamid wird nach Zugabe von 100 mg Palladium-Kohle (10%) während 4 Stunden bei Raumtemperatur und Normaldruck hydriert. Zur Aufarbeitung wird die Lösung nach Abfiltrieren des Katalysators im Hochvakuum auf 2 ml konzentriert und das Produkt mit 40 ml peroxidfreiem Äther ausgefällt, abfiltriert und im Vakuum getrocknet. Das Rohprodukt wird ohne weitere Reinigung der nächsten Stufe 3.4 (Cyclisierung) unterzogen.

Stufe 3.4

Asn–Phe–Phe–(D–trp)–Lys(Boc)–Thr(But)–Phe–(β-Cyclohexyl)Gaba

Eine Lösung von 836 mg rohem
H–Asn–Phe–Phe–(D–trp)–Lys(Boc)–Thr(But)–Phe–(d,l-β-Cyclohexyl)Gaba-OH
(Stufe 3.3), 860 mg N-Hydroxybenzotriazol und 1,31 g DCCI in 640 ml Dimethylformamid wird während 20 Stunden bei 50° gehalten. Zur Aufarbeitung wird das Lösungsmittel im Hochvakuum bei ca. 30° abgedampft und der Rückstand mit 20 ml Äthylacetat verrieben. Der ausgefallene Dicyclohexylharnstoff wird durch Abfiltrieren beseitigt, das Filtrat mit Äthylacetat auf 200 ml verdünnt, dreimal mit je 50 ml 1 N wässeriger Oxalsäure und dann mit Wasser bis zur Neutralität gewaschen, über Natriumsulfat getrocknet und im Vakuum eingedampft. Das Rohprodukt wird zur Reinigung der Gegenstromverteilung im System Methanol-Wasser-Chloroform-Tetrachlorkohlenstoff (2700 : 675 . 900 : 1575 Vol.-Teile) über 400 Stufen unterzogen. Die in den Elementen 81 bi 108 befindlichen Phasen (K = 0,49) dann das andere (Isomer B). Die Phasen der entsprechenden Elemente werden vereinigt und im Vakuum eingedampft. Der Rückstand wird in 20 ml tert.-Butanol gelöst und lyophilisiert, womit jeweils ein dünnschichtchromatographisch einheitliches Diastereomeres der obigen gemeinsamen Formel resultiert.

DC: [Chloroform-Methanol (85 : 15)] Isomer A: $R_f$ 0,51, Isomer B. $R_f$ 0,42.

Beispiel 4A

Asn–Phe–Phe–(D–trp)–Lys–Thr–Phe–[β-(3-Phenoxyphenyl)]Gaba -Isomer A

280 mg geschütztes Octapeptid der Formel

Asn–Phe–Phe–(D–trp)–Lys(Boc)–Thr(But)–Phe–[β-(3-Phenoxyphenyl)]Gaba -Isomer A

wird bei 5° unter $N_2$ in 2,8 ml eines Gemisches aus 89 Vol.-% Trifluoressigsäure, 10 Vol.-% Wasser und 1 Vol.-% Thioglykolsäure gelöst, die Lösung sofort auf 25° erwärmt und nach 50 Minuten bei dieser Temperatur mit 25 ml peroxidfreiem Äther ausgefällt. Das resultierende rohe Trifluoracetat des Endprodukts wird abfiltriert, im Vakuum getrocknet, in 5 ml 1 N Essigsäure gelöst und durch 15 ml Anionenaustauscher, z.B. AG® 1–X8 (ein Produkt von Bio-Rad Raboratories, Richmond, Calif., USA), in Acetatform, filtriert; das Eluat wird lyophilisiert.

Die erhaltene Titelverbindung ist in drei Systemen dünnschichtchromatographisch einheitlich.

DC: System 52. $R_f$ 0,49
111B: $R_f$ 0,40
157F: $R_f$ 0,53

CD: in 1%iger wässriger Essigsäure: λ/mol.Ellipt. [nm/Grad.cm².dmol$^{-1}$]: 210/−37430 (min); 222/−534 (max); 230/−15506 (min).

Beispiel 4B

Asn–Phe–Phe–(D–trp)–Lys–Thr–Phe–[β-(3-Phenoxyphenyl)]Gaba -Isomer B

268 mg geschütztes Octapeptid der Formel

Asn–Phe–Phe–(D–trp)–Lys(Boc)–Thr(But)–Phe–[β-(3-Phenoxyphenyl)]Gaba -Isomer B

wird bei 5° unter $N_2$ in 2,7 ml eines Gemisches aus 89 Vol.-% Trifluoressigsäure, 10 Vol.-% Wasser und 1 Vol.-% Thioglykolsäure gelöst, die Lösung sofort auf 25° erwärmt und nach 50 Minuten bei dieser Temperatur mit 30 ml peroxidfreiem Äther ausgefällt. Das resultierende rohe Trifluoracetat des Endprodukts wird abfiltriert, im Vakuum getrocknet, in 5 ml 1 N Essigsäure gelöst und durch 15 ml Anionenaustauscher, z.B. AG® 1–X8 (ein Produkt von Bio-Rad Raboratories, Richmond, Calif., USA), in Acetatform, filtriert; das Eluat wird lyophilisiert.

Die erhaltene Titelverbindung ist in drei Systemen dünnschichtchromatographisch einheitlich.

DC: System 52: $R_f$ 0,49
111B: $R_f$ 0,38
157F: $R_f$ 0,52.

CD. in 1%iger wässriger Essigsäure: λ/mol.Ellipt. [nm/Grad.cm².dmol$^{-1}$]: 220/+30354 (max); 232,5/−13811 (min).

Der peptidische Ausgangsstoff der Beispiele 4A und 4B ist folgendermassen erhältlich:

Stufe 4.1

d,1-H-[β-(3-Phenoxyphenyl)]Gaba-OBzl–p-toluolsulfonat

Ein Gemisch von 0,500 g d,l-4-Amino-3-(3-phenoxyphenyl)-buttersäurehydrochlorid und 0,308 g p-Toluolsulfonsäure-monohydrat in 0,85 ml Benzylalkohol und 70 ml Benzol wird bei Normaldruck langsam destilliert, bis während 3 Stunden insgesamt 30 ml einer Fraktion, Sdp. 70–90°, aufgefangen wird. Die klare Reaktionslösung wird im Wasserstrahlvakuum und dann im Hochvakuum bei ca. 60° auf 3 ml konzentriert. Der ausgefallene Kristallbrei wird mit 15 ml Äther verrührt, die Kristalle abfiltriert und mit 10 ml Äther gewaschen. Zur weiteren Reinigung wird dieses Material mit 10 ml Äther bei Raumtemperatur während einer Stunde verrührt, abfiltriert, mit Äther gewaschen und im Vakuum getrocknet.

DC: [Chloroform-Methanol-Wasser (14 : 6 . 1)]$R_f$ 0,61
[Chloroform-Methanol (85 : 15)] $R_f$ 0,30.

Stufe 4.2

Z–Asn–Phe–Phe–(D–trp)–Lys(Boc)–Thr(But)–Phe–[d,l-β-(3-Phenoxyphenyl)]Gaba-OBzl

Ein Gemisch von 819 mg

Z–Asn–Phe–Phe–(D–trp)–Lys(Boc)–Thr(But)–Phe–OH

(vgl. Stufe 1.2) und 341 mg

d,l-4-Amino-3-(3-phenoxyphenyl)-buttersäure-benzylester-p-toluolsulfonat

(Stufe 4.1) in 6 ml Dimethylformamid wird mit 0,071 ml N-Methylmorpholin, 95 mg N-Hydroxybenzotriazol und 171 mg DCCI versetzt und 20 Stunden bei Raumtemperatur belassen. Zur Aufarbeitung wird vom ausgefallenen DCC abzentrifugiert, der Überstand mit 20 ml Wasser versetzt und abfiltriert. Der gewonnene Feststoff wird zur weiteren Reinigung mit 3 ml Methanol während 10 Minuten verrührt, die Suspension auf 0° abgekühlt, das reine Produkt abfiltriert und im Vakuum getrocknet.

DC: [Chloroform-Methanol (85 . 15)] $R_f$ 0,76
[Chloroform-Methanol-Wasser (14 . 6 : 1)] $R_f$ 0,94.

Stufe 4.3

H–Asn–Phe–Phe–(D–trp)–Lys(Boc)–Thr(But)–Phe–[d,1-β-(3-Phenoxyphenyl)]GabaOH

Eine Lösung von 943 mg

Z–Asn–Phe–Phe–(D–trp)–Lys(Boc)–Thr(But)–Phe–[d,l-β-(3-Phenoxyphenyl)]GabaOBzl

(Stufe 4.2) in 30 ml Dimethylformamid wird nach Zugabe von 100 mg Palladium-Kohle (10%) während 4 Stunden bei Raumtemperatur und Normaldruck hydriert. Zur Aufarbeitung wird die Lösung nach Abfiltrieren des Katalysators im Hochvakuum auf 2 ml konzentriert und das Produkt mit 40 ml peroxidfreiem Äther ausgefällt, abfiltriert

und im Vakuum getrocknet. Das Rohprodukt wird ohne weitere Reinigung der nächsten Stufe 4.4 (Cyclisierung) unterzogen.

Stufe 4.4

Asn–Phe–Phe–(D–trp)–Lys(Boc)–Thr(But)–
Phe–[β-(3-Phenoxyphenyl)]Gaba

Eine Lösung von 811 mg rohem
H–Asn–Phe–Phe–(D–trp)–Lys(Boc)–Thr(But)–
Phe–[(d,l-β-(3-Phenoxyphenyl)]Gaba-OH
(Stufe 4.3), 784 mg N-Hydroxybenzotriazol und 1,20 g DCCI in 580 ml Dimethylformamid wird während 20 Stunden bei 50° gehalten. Zur Aufarbeitung wird das Lösungsmittel im Hochvakuum bei ca. 30° abgedampft und der Rückstand mit 20 ml Äthylacetat verrieben. Der ausgefallene Dicyclohexylharnstoff wird durch Abfiltrieren beseitigt, das Filtrat mit Äthylacetat auf 200 ml verdünnt, dreimal mit je 50 ml 1 N wässeriger Oxalsäure und dann mit Wasser bis zur Neutralität gewaschen, über Natriumsulfat getrocknet und im Vakuum eingedampft. Das Rohprodukt wird zur Reinigung der Gegenstromverteilung im System Methanol-Wasser-Chloroform-Tetrachlorkohlenstoff (2700 : 675 : 900 : 1575 Vol.-Teile) über 600 Stufen unterzogen. Die in den Elementen 80 bis 113 befindlichen Phasen (K = 0,17) enthalten eines der beiden Diastereomeren (Isomer A), die in den Elementen 125–167 befindlichen Phasen (K = 0,31) dann das andere (Isomer B). Die Phasen der entsprechenden Elemente werden vereinigt und im Vakuum eingedampft. Der Rückstand wird in 20 ml tert.-Butanol gelöst und lyophilisiert, womit jeweils ein dünnschichtchromatographisch einheitliches Diastereomeres der obigen gemeinsamen Formel resultiert.

DC: [Chloroform-Methanol (85 : 15)] Isomer A: $R_f$ 0,60; Isomer B: $R_f$ 0,51
[Chloroform-Methanol-Wasser (14 : 6 : 1)] Isomer A: $R_f$ 0,89;
Isomer B: $R_f$ 0,83.

Beispiel 5

Asn–Phe–Phe–(5-F–D–trp)–Lys–Thr–Phe–[β-(1-Naphthyl)]Gaba -Isomer B

(5-F–D–trp = 5-Fluor-D-tryptophyl)

147 mg geschütztes Octapeptid der Formel

Asn–Phe–Phe–(5-F–D–trp)–Lys(Boc)–
Thr(But)–Phe–[β-(1-Naphthyl)]Gaba -Isomer B

wird bei 5° unter $N_2$ in 2,5 ml eines Gemisches aus 89 Vol.-% Trifluoressigsäure, 10 Vol.-% Wasser und 1 Vol.-% Thioglykolsäure gelöst, die Lösung sofort auf 25° erwärmt und nach 50 Minuten bei dieser Temperatur mit 15 ml peroxidfreiem Äther ausgefüllt. Das resultierende rohe Trifluoracetat des Endprodukts wird abfiltriert, im Vakuum getrocknet, in 5 ml 1 N Essigsäure gelöst und durch 15 ml Anionenaustauscher, z.B. AG® 1–X8 (ein Produkt von Bio-Rad Laboratories, Richmond, Calif., USA), in Acetatform, filtriert. Das Eluat wird im Vakuum eingedampft und der Rückstand der Gegenstromverteilung im System n-Butanol-Essigsäure-Wasser (4 : 1 : 5) über 230 Stufen unterzogen. Die in den Elementen 230 bis 274 enthaltenen Phasen (K = 13,7) werden gesammelt, im Vakuum eingedampft und aus tert.-Butanol-Wasser (1 : 1) lyophilisiert.

Die erhaltene Titelverbindung ist in drei Systemen dünnschichtchromatographisch einheitlich.

DC. System 52: $R_f$ 0,50
111B: $R_f$ 0,45
157F: $R_f$ 0,55.

Der peptidische Ausgangsstoff ist folgendermassen erhältlich:

Stufe 5.1

Z–Asn–Phe–Phe–(5-F–D–trp)–Lys(Boc)–
Thr(But)–Phe–[d,l-β-(1-Naphthyl)]Gaba-OBzl

Ein Gemisch von 411 mg Z–Asn–Phe–Phe–(5-F–D–trp)–Lys(Boc)–Thr(But)–Phe–OH (vgl. Stufe 11.5, Beispiel 11 der eingangs angegebenen Europäischen Patentanmeldung) und 172 mg d,l-4-Amino-3-(1-naphthyl)-buttersäure-benzylester-p-toluolsulfonat (Stufe 2.1) in 3 ml Dimethylformamid wird mit 0,039 ml N-Methylmorpholin, 47 mg N-Hydroxybenzotriazol und 85 mg DCCI versetzt und 20 Stunden bei Raumtemperatur belassen. Zur Aufarbeitung wird vom ausgefallenen DCC abzentrifugiert, der Überstand mit 10 ml Wasser versetzt und abfiltriert. Der gewonnene Feststoff wird zur weiteren Reinigung mit 2 ml Methanol während 10 Minuten verrührt, die Suspension auf 0° abgekühlt, das reine Produkt abfiltriert und im Vakuum getrocknet.

DC: [Chloroform-Methanol (85 : 15)] $R_f$ 0,67
[Chloroform-Methanol-Wasser (14 : 6 : 1)] $R_f$ 0,87.

Stufe 5.2

H–Asn–Phe–Phe–(5-F–D–trp)–Lys(Boc)–
Thr(But)–Phe–[d,l-β-(1-Naphthyl)]
Gaba–OH

Eine Lösung von 421 mg
Z–Asn–Phe–Phe–(5-F–D–trp)–Lys(Boc)–Thr(But)–
Phe–[d,l-β-(1-Naphthyl)]Gaba-OBzl
(Stufe 5.1) in 15 ml Dimethylformamid wird nach Zugabe von 50 mg Palladium-Kohle (10%) während 4 Stunden bei Raumtemperatur und Normaldruck hydriert. Zur Aufarbeitung wird die Lösung nach Abfiltrieren des Katalysators im Hochvakuum auf 2 ml konzentriert und das Produkt mit 40 ml peroxidfreiem Äther ausgefällt, abfiltriert und im Vakuum getrocknet. Das Rohprodukt wird ohne weitere Reinigung der nächsten Stufe 5.3 (Cyclisierung) unterzogen.

Stufe 5.3

Asn–Phe–Phe–(5-F–D–trp)–Lys(Boc)–Thr-
(But)–Phe–[β-(1-Naphthyl)]Gaba

Eine Lösung von 357 mg rohem

H–Asn–Phe–Phe–(5-F–D–trp)–Lys(Boc)–Thr(But)–
Phe–[d,l-β-(1-Napthyl)]Gaba-OH

(Stufe 5.2), 355 mg N-Hydroxybenzotriazol und 543 mg DCCI in 260 ml Dimethylformamid wird während 20 Stunden bei 50° gehalten. Zur Aufarbeitung wird das Lösungsmittel im Hochvakuum bei ca. 30° abgedampft und der Rückstand mit 20 ml Äthylacetat verrieben. Der ausgefallene Dicyclohexylharnstoff wird durch Abfiltrieren beseitigt, das Filtrat mit Äthylacetat auf 200 ml verdünnt, dreimal mit je 50 ml 1 N wässeriger Oxalsäure und dann mit Wasser bis zur Neutralität gewaschen, über Natriumsulfat getrocknet und im Vakuum eingedampft. Das Rohprodukt wird zur Reinigung der Gegenstromverteilung im System Methanol-Wasser-Chloroform-Tetrachlorkohlenstoff (2700 : 675 : 900 : 1575 Vol.-Teile) über 480 Stufen unterzogen. Die in den Elementen 75 bis 114 befindlichen Phasen (K = 0,24) enthalten eines der beiden Diastereomeren (Isomer A), die in den Elementen 161–186 befindlichen Phasen (K = 0,50) dann das andere (Isomer B). Die Phasen der entsprechenden Elemente werden vereinigt und im Vakuum eingedampft. Der Rückstand wird in 20 ml tert.-Butanol gelöst und lyophilisiert, womit jeweils ein dünnschichtchromatographisch einheitliches Diastereomeres der obigen gemeinsamen Formel resultiert.

DC: [Chloroform-Methanol (85 : 15)] Isomer A. $R_f$ 0,54; Isomer B. $R_f$ 0,45.
[Chloroform-Methanol-Wasser (14 : 6 : 1)] Isomer A. $R_f$ 0,90;
Isomer B: $R_f$ 0,85.

Beispiel 6

Asn–Phe–Phe–(D–trp)–Lys–Thr–Phe–[β-(4-Chlor-1-naphthyl)]Gaba -Isomer B

178 mg geschütztes Octapeptid der Formel

Asn–Phe–Phe–(D–trp)–Lys(Boc)–Thr(But)–Phe–[β-(4-Chlor-1-naphthyl)]Gaba -Isomer B

wird in genau gleicher Weise, wie in Beispiel 1 B beschrieben, verarbeitet. Die Reinigung des Produkts erfolgt durch Gegenstromverteilung im System tert.-Butanol-Toluol-Methanol-Puffer (800 : 800 : 340 : 1140) (Puffer enthaltend 2,2 g Ammoniumacetat und 1,6 ml Eisessig in 1 Liter Wasser) über 770 Stufen. Die reines Isomer B enthaltenden Phasen (K = 1,1) werden im Vakuum eingedampft und der Rückstand aus tert.-Butanol-Wasser lyophilisiert.

DC: System 52: $R_f$ 0,45
157C: $R_f$ 0,24.

In analoger Weise erhält man auch

Asn–Phe–Phe–(D–trp)–Lys–Thr–Phe–[β-(4-Nitrophenyl)]Gaba -Isomer B,

K = 0,33 (im obigen Lösungsmittelsystem),
DC: System 157C: $R_f$ 0,28.

Die peptidischen Ausgangsstoffe sind folgendermassen erhältlich:

Stufe 6.1

H–Asn–Phe–Phe–(D–trp)–Lys(Boc)–Thr(But)–Phe–OH

Eine Lösung von 3,1 g

Z–Asn–Phe–Phe–(D–trp)–Lys(Boc)–Thr(But)–Phe–OH

(hergestellt nach Stufe 1.7, Beispiel 1 der eingangs zitierten Europäischen Patentanmeldung) in 100 ml Dimethylformamid wird nach Zugabe von 300 mg Palladium-Kohle (10%) während 2 Stunden hydriert. Nach Abfiltrieren des Katalysators wird die Lösung im Hochvakuum auf 20 ml eingeengt und ohne weitere Reinigung für die nächste Stufe 6.2 verwendet.

Stufe 6.2

$N^\alpha$-2-Trimethylsilyläthoxycarbonyl–Asn–Phe–Phe–(D–trp)–Lys(Boc)–Thr(But)–Phe–OH

Die in Stufe 6.1 erhaltene Dimethylformamidlösung des geschützten Heptapeptids wird mit 940 mg 2-Trimethylsilyläthyl-succinimidylcarbonat ($Me_3SiCH_2CH_2OCOON<$ $COCH_2$ | $COCH_2$ ) und 0,27 ml N-Methylmorpholin versetzt und 45 Minuten bei Raumtemperatur reagieren gelassen. Nach Zugabe von weiteren 0,13 ml N-Methylmorpholin wird nochmals 2½ Stunden reagieren gelassen. Das Produkt wird durch Eintropfen in 100 ml 0,2 N Salzsäure bei 0° gefällt und zur Reinigung je dreimal mit Äthylacetat und mit Acetonitril zerrieben.

DC: System 157A: $R_f$ 0,29.

Stufe 6.3

$N^\alpha$-2-Trimethylsilyläthoxycarbonyl–Asn–Phe–Phe–(D–trp)–Lys(Boc)–Thr(But)–Phe–[d,l-β-(4-Chlor-1-naphthyl)]Gaba-OH

Zu einer Lösung von 1,35 g

$N^\alpha$-2-Trimethylsilyl-äthoxycarbonylAsn–Phe–Phe–(D–trp)–Lys(Boc)–Thr(But)–Phe–OH

(Stufe 6.2) und 180 mg N-Hydroxysuccinimid in 2 ml Dimethylformamid wird bei 0° eine Lösung von 216 mg DCCI in 0,0 ml Dimethylformamid zugegeben und 4½ Stunden reagieren gelassen (Teil 1). In der Zwischenzeit werden 304 mg d,l-4-Amino-3-(4-chlor-1-naphthyl)-buttersäure in 0,6 ml 2 N Benzyltrimethylammoniumhydroxid in Methanol unter Zugabe von 10 ml Dimethylformamid und wenig Wasser gelöst und im Hochvakuum wieder auf 3,5 ml eingeengt. Die trübe Lösung wird bei 0° zum obigen Teil 1 gegeben, noch zweimal mit 0,2 ml Dimethylformamid nachgewaschen und über Nacht bei Raumtemperatur reagieren gelassen. Das Produkt wird durch Eintropfen in 100 ml eiskalte 0,2 N Salzsäure ausgefällt, abfiltriert und mit Wasser gewaschen. Nach dem Trocknen wird noch je dreimal mit Äthylacetat und Acetonitril zerrieben.

DC: System 157A: $R_f$ 0,39 und 0,41 (Diastereomere).

In analoger Weise, ausgehend von d,l-4-Amino-3-(4-nitro-phenyl)-buttersäure wird

$N^{\alpha}$-2-Trimethylsilyläthoxycarbonyl–Asn–Phe–
Phe–(D–trp)–Lys(Boc)–Thr(But)–Phe–
[d,l-β-(4-Nitrophenyl)]Gaba-OH
erhalten;
DC: System 157A: $R^f$ 0,37.

Stufe 6.4
H–Asn–Phe–Phe–(D–trp)–Lys(Boc)–Thr(But)–
Phe–[d,l-β-(4-Chlor-naphthyl)]Gaba–
OH,Hydrochlorid

Eine Lösung von 1,55 g
2-Trimethylsilyläthoxycarbonyl–Asn–Phe–
Phe–(D–trp)–Lys(Boc)–Thr(But)–
Phe–[d,l-β-(4-Chlor-naphthyl)]Gaba-OH
(Stufe 6.3) in 4,2 ml Dimethylformamid wird bei 0° innerhalb 2 Minuten mit 8,6 ml einer 0,59 M Lösung von Tetraäthylammoniumfluorid in Dimethylsulfoxid versetzt und 3 Stunden bei 30° reagieren gelassen. Das Produkt wird durch Eintropfen des Reaktionsgemisches in 200 ml eiskalte 0,1 N Salzsäure ausgefällt. Die Fällung wird abzentrifugiert, mit eiskaltem Wasser gewaschen, in tert.-Butanol gelöst und lyophilisiert. Der Rückstand wird in 3 ml Dimethylformamid gelöst und mit Wasser wieder gefällt. Das durch Zentrifugieren isolierte Produkt wird noch mehrmals mit Wasser gewaschen und im Hochvakuum getrocknet.
DC: System 157A: $R_f$ 0,09 und 0,12 (Diastereomere).

In analoger Weise wird aus entsprechendem Ausgangsstoff (vgl. Stufe 6.3) das
H–Asn–Phe–Phe–(D–trp)–Lys(Boc)–Thr(But)–
Phe–[d,l-β-(4-Nitrophenyl)]Gaba-OH.
Hydrochlorid
erhalten:
DC: System 157C: $R_f$ 0,53 und 0,55 (Diastereomere).

Stufe 6.5

└Asn–Phe–Phe–(D–trp)–Lys(Boc)–Thr(But)–
Phe–[β-(4-Chlor-1-naphthyl)]Gaba┘-Isomer B.

Eine Lösung von 1,25 g
HCl.H–Asn–Phe–Phe–(D–trp)–Lys(Boc)–Thr(But)–
Phe–[d,l-β-(4-Chlor-1-naphthyl)]Gaba-OH
(Stufe 6.4), 1,33 g N-Hydroxybenzotriazol-monohydrat, 0,1 ml N-Methyl-morpholin und 1,8 g DCCl in 800 ml Dimethylformamid wird während 18 Stunden bei 50° gehalten. Zur Aufarbeitung wird 1,1 g Oxalsäure zugegeben, die Lösung im Hochvakuum stark eingeengt und der ausgefallene Dicyclohexylharnstoff durch Abfiltrieren beseitigt. Das Filtrat wird durch Eintropfen in verdünnte Natriumhydrogencarbonat-Lösung gefällt. Das Rohprodukt wird zur Reinigung der Gegenstromverteilung im System Methanol-Wasser-Chloroform-Tetrachlorkohlenstoff (2700 : 675 : 900 : 1575 Vol.-Teile) über 560 Stufen unterzogen. Das in den Elementen 154–228 befindliche Produkt (K = 0,43) wird in der üblichen Weise isoliert – Isomer B: DC. System 157A: $R_f$ 0,33.

In analoger Weise erhält man aus entsprechendem linearem Ausgangsstoff (vgl. Stufe 6.4) durch Cyclisierung und anschliessende Gegenstromverteilung in demselben Lösungsmittelsystem das

└Asn–Phe–Phe–(D–trp)–Lys(Boc)–Thr(But)–
Phe–[β-(4-Nitrophenyl)]Gaba┘ Isomer B: K = 0,8
DC: System 157A: $R_f$ 0,27.

In den nachstehenden Beispielen 7–13 wird die Herstellung pharmazeutischer Applikationsformen illustriert. Die Bezeichnung «Wirkstoff» bezieht sich auf die erfindungsgemäss erhältlichen Endstoffe der Formel I, insbesondere auf diejenigen von Beispielen 1–6 und in erster Linie auf das [D–trp$^8$; β-(1-Naphthyl)Gaba$^{12}$]-cyclo-somatostatin(5–12)-octapeptid-Isomer B vom Beispiel 2B und [D–trp$^8$; β-(3-phenoxyphenyl)Gaba$^{12}$]-cyclo-somatostatin(5–12)-octapeptid vom Beispiel 4B.

Beispiel 7

A) Eine Injektionslösung enthaltend 2,0 mg Wirkstoff wird folgendermassen erhalten: Man löst 1,0 mg Eisessig, 0,8 mg Natriumacetat, 8,0 mg Natriumchlorid und 2,0 mg Wirkstoff in 0,7 ml destilliertem Wasser und füllt mit destilliertem Wasser auf 1 ml auf. Die Lösung wird bei 120 °C 20 Minuten lang im Autoklav erhitzt. Das pH nach der Sterilisation ist 4,5.

B) Eine Injektionslösung enthaltend 0,5 mg des Wirkstoffes wird folgendermassen erhalten:

Man löst in 0,7 ml physiologischer Natriumchloridlösung 0,5 mg Wirkstoff und säuert die Lösung mit 0,1-N Salzsäure auf pH 4,0 an. Mit destilliertem Wasser wird auf 1 ml aufgefüllt und sterilfiltriert.

Beispiel 8

A) Eine Gelatine-enthaltende Injektionslösung enthaltend 0,1 mg Wirkstoff wird folgendermassen erhalten:

Eine sterilfiltrierte wässrige Lösung des Wirkstoffes wird unter Erwärmen mit einer sterilen Gelatinelösung, welche als Konservierungsmittel Phenol enthält, unter aseptischen Bedingungen vermischt, sodass 1,0 ml Lösung folgende Zusammensetzung hat:

| | |
|---|---|
| Wirkstoff | 0,1 mg |
| Gelatine | 150,0 mg |
| Phenol | 4,7 mg |
| dest. Wasser bis zu | 1,0 ml |

Die Mischung wird aseptisch in Vials zu 1,0 ml abgefüllt.

B) Eine analoge Injektionslösung enthaltend 0,5 mg des Wirkstoffes erhält man in gleicher Weise wie oben angegeben, indem man eine Mischung der folgenden Zusammensetzung herstellt:

| | |
|---|---|
| Wirkstoff | 0,5 mg |
| Gelatine | 280,0 mg |
| Phenol | 5,0 mg |
| dest. Wasser bis zu | 1,0 ml |

Die Mischung wird aseptisch in Vials zu 1,0 ml abgefüllt.

Beispiel 9

Präparat enthaltend 0,5 mg Wirkstoff als sterile Trockensubstanz zur Injektion wird folgendermassen erhalten. Man löst 0,5 mg Wirkstoff in 1 ml einer wässrigen Lösung von 20 mg Mannit. Die Lösung wird sterilfiltriert und unter aseptischen Bedingungen in eine 2-ml-Ampulle gefüllt, tiefgekühlt und lyophilisiert. Vor dem Gebrauch wird das Lyophilisat in destilliertem Wasser gelöst. Die Lösung wird intramuskulär oder intravenös angewendet.

Beispiel 10

Injektionspräparat enthaltend den Wirkstoff als Polyphosphat-Suspension wird folgendermassen erhalten:

A) Mit 1,0 mg Wirkstoff: Man mischt eine Lösung von 1,0 mg Wirkstoff und 9,0 mg Natriumchlorid in 0,5 ml destilliertem Wasser mit einer Lösung von 2 mg Natriumpolyphosphat (Calgon N®) in 0,5 ml destilliertem Wasser. Die erhaltene Suspension hat folgende Zusammensetzung:

| | |
|---|---|
| Wirkstoff | 1,0 mg |
| Natriumpolyphosphat (Calgon N®) | 2,0 mg |
| Natriumchlorid | 9,0 mg |
| dest. Wasser bis zu | 1,0 ml |

Die Suspension weist ein pH von 6,9 auf. Sie ist für intramuskuläre Anwendung geeignet.

B) Mit 0,5 mg Wirkstoff:

In gleicher Weise wie oben angegeben wird eine Suspension der folgenden Zusammensetzung hergestellt:

| | |
|---|---|
| Wirkstoff | 0,5 mg |
| Natriumpolyphosphat (Calgon 322®) | 1,0 mg |
| Natriumchlorid | 9,0 mg |
| dest. Wasser bis zu | 1,0 ml |

Das pH der Suspension beträgt 5,9.

Beispiel 11

Injektionspräparat enthaltend 0,3 mg Wirkstoff in Form eines öligen Aluminiumstearat-Gels.

Ein 2%iges Aluminiumstearat-Gel wird auf übliche Art und Weise durch Suspendieren von 1,0 g Aluminiummonostearat in 49,0 g Erdnussöl und anschliessendes Erwärmen auf 130 °C während 10 Min. hergestellt. 15,0 mg Wirkstoff werden in 0,3 g obigem Aluminiumstearat-Gel suspendiert, homogenisiert und mit der restlichen Menge Aluminiumstearat-Gel verdünnt. Das so erhaltene Gel hat folgende Zusammensetzung:

| | |
|---|---|
| Wirkstoff | 0,3 mg |
| Aluminiummonostearat | 20,0 mg |
| Erdnussöl bis zu | 1,0 ml |

Die ölige Aluminiumstearat-Gel-Suspension ist für intramuskuläre Anwendung geeignet.

Beispiel 12

Injektionspräparat enthaltend 0,5 mg Wirkstoff als eine Depot-Suspension mit Dextransulfat.

Man löst in 0,4 ml destilliertem Wasser 0,36 mg Essigsäure, 1,9 mg Natriumacetat-trihydrat, 8,0 mg Natriumchlorid und 0,5 mg Wirkstoff und füllt mit destilliertem Wasser auf 0,5 ml auf. Zu dieser Lösung wird unter Rühren 0,5 ml einer 0,1%igen Lösung von Dextransulfat (Molekulargewicht 500 000) gegeben, womit sich eine homogene Ausfällung bildet. Die erhaltene Suspension hat folgende Zusammensetzung:

| | |
|---|---|
| Wirkstoff | 0,50 mg |
| Dextransulfat MG 500 000 | 0,50 mg |
| Essigsäure 100% | 0,36 mg |
| Natriumacetat-trihydrat | 1,90 mg |
| Natriumchlorid | 8,00 mg |
| dest. Wasser bis zu | 1,00 ml |

Die wässrige Suspension ist für intramuskuläre und subcutane Injektion geeignet.

Beispiel 13

Nasal-Spray

30 mg feingemahlener Wirkstoff wird in einer Mischung von 75 mg Benzylalkohol und 1,395 g eines Gemisches von halbsynthetischen Glyceriden gesättigter Fettsäuren mit 8–12 Kohlenstoffatomen (z.B. Miglyol® 812) suspendiert. Mit dieser Suspension werden Aluminium-Monoblocdosen (Gehalt 10 ml) eingefüllt, mit einem Dosierventil verschlossen und mit 6,0 g eines Gemisches von Dichloridfluormethan und 1,2-Dichlor-1,1,2,2-tetrafluoräthan (z.B. Freon® 12/114) im Vol.-Verhältnis 40 : 60 unter Stickstoffdruck eingefüllt. Die Aluminiumdose mit einem Füllgewicht von insgesamt 7,5 g enthält 100 Einzeldosen à 0,3 mg Wirkstoff. Die Sprühdose ist mittels des Ventils so eingestellt, dass bei einmaligem Druck eine Einzeldosis versprüht wird.

In gleicher Weise werden Nasensprays hergestellt, welche statt Miglyol® die gleiche Gewichtsmenge Isopropylmyristat oder Isopropylpalmitat oder eine Mischung von Glycerin- und Polyoxyäthylenglycolestern von Fettsäuren mit 8 und 10 Kohlenstoffatomen (z.B. Labrafac® WL 1219) enthalten.

**Patentansprüche für die Vertragsstaaten: BE, CH, DE, FR, GB, IT, LI, LU, NL, SE**

(für alle benannten Länder ausser Österreich)

1. Verfahren zur Herstellung von cyclischen Octapeptiden der Formel

$$\overline{\underline{|\text{Asn–Phe–Phe–trp–Lys–Thr–Phe–Gaba(Ar)}|}}$$

$$\begin{array}{cccccccc} 5 & 6 & 7 & 8 & 9 & 10 & 11 & 12 \end{array} \qquad (I)$$

worin trp für L–Trp oder D–Trp, in welchen der Benzolring durch ein Halogenatom substituiert sein kann, und Gaba(Ar) für den Rest einer durch ein cyclisches Hydrocarbyl substituierten $\gamma$-Aminobuttersäure steht, sowie Säureadditionssalzen und Komplexen davon, dadurch gekennzeichnet, dass man ein lineares Peptid der Formel

$$\text{H–[II']-C} \qquad (III)$$

worin II′ einen der unten definierten Formel II entsprechenden Rest darstellt, in welchem die amidische Bindung zwischen zwei beliebigen benachbarten Aminosäureresten des Peptidringes

unterbrochen ist, und C für eine freie Hydroxylgruppe, eine durch eine Aktivierungsgruppe abgewandelte Hydroxylgruppe oder die Hydrazinogruppe $-NH-NH_2$ steht, cyclisiert, und in einer erhaltenen Verbindung der Formel

└Asn–Phe–Phe–trp–Lys(A)–Thr(B)–

Phe–Gaba(Ar)┘ (II)

worin Gaba(Ar) und trp die obgenannten Bedeutungen haben, A eine ε-Aminoschutzgruppe oder Wasserstoff und B eine Hydroxylschutzgruppe oder Wasserstoff bedeutet, wobei nur eines der Symbole A und B für Wasserstoff stehen kann, die Schutzgruppe(n) abspaltet und, wenn erwünscht, aus einem erhaltenen Säureadditionssalz die entsprechende Base freisetzt und/oder eine erhaltene Base in ein Säureadditionssalz davon umwandelt.

2. Verfahren gemäss Anspruch 1, dadurch gekennzeichnet, dass man ein Octapeptid der Formel I, worin trp für L–Trp oder D–Trp und Gaba(Ar) für den Rest einer durch ein cyclisches Hydrocarbyl substituierten γ-Aminobuttersäure steht, oder ein Säureadditionssalz oder einem Komplex davon herstellt.

3. Ein cyclisches Octapeptid der Formel

└Asn–Phe–Phe–trp–Lys–Thr–Phe–Gaba(Ar)┘

5 6 7 8 9 10 11 12 (I)

worin trp für L–Trp oder D–Trp, in welchen der Benzolring durch ein Halogenatom substituiert sein kann, und Gaba(Ar) für den Rest einer durch ein cyclisches Hydrocarbyl substituierten γ-Aminobuttersäure steht, sowie pharmazeutisch verwendbare Säureadditionssalze und Komplexe davon.

4. Eine Verbindung gemäss Anspruch 3, worin trp für L–Trp oder D–Trp und Gaba(Ar) für den Rest einer durch ein cyclisches Hydrocarbyl substituierten γ-Aminobuttersäure steht, sowie pharmazeutisch verwendbare Säureadditionssalze und Komplexe davon.

5. Eine Verbindung gemäss Anspruch 4, worin trp für D–Trp und Gaba(Ar) für den Rest der 4-Amino-3-cyclohexylbuttersäure, 4-Amino-3-phenylbuttersäure, 4-Amino-3-(1-naphthyl)-buttersäure oder 4-Amino-3-(2-naphthyl)-buttersäure steht, in Form eines Diastereomerengemisches oder eines individuellen Diastereomeren, sowie pharmazeutisch verwendbare Säureadditionssalze und Komplexe davon.

6. Eine Verbindung gemäss Anspruch 3, worin trp für D–Trp und Gaba(Ar) für den Rest der
4-Amino-3-(4-fluorphenyl)-buttersäure,
4-Amino-3-(4-nitrophenyl)-buttersäure,
4-Amino-3-(4-chlor-1-naphthyl)-buttersäure oder
4-Amino-3-(3-phenoxyphenyl)-buttersäure
steht, in Form eines Diastereomerengemisches oder eines individuellen Diastereomeren, sowie pharmazeutisch verwendbare Säureadditionssalze und Komplexe davon.

7. [D–Trp$^8$; β-Cyclohexyl-Gaba$^{12}$]-cyclo-somatostatin(5–12)-octapeptid, in Form eines Diastereomerengemisches oder eines individuellen Diastereomeren, sowie pharmazeutisch verwendbare Säureadditionssalze und Komplexe davon.

8. [D–Trp$^8$; β-(3-Phenoxyphenyl)-Gaba$^{12}$]-cyclo-somatostatin(5–12)-octapeptid, in Form eines Diastereomerengemisches oder eines individuellen Diastereomeren, sowie pharmazeutisch verwendbare Säureadditionssalze und Komplexe davon.

9. Eine Verbindung gemäss einem der Ansprüche 3–8 in Form des Isomeren B.

10. [D–Trp$^8$; β-(1-Naphthyl)-Gaba$^{12}$]-cyclo-somatostatin(5–12)-octapeptid, in Form eines Diastereomerengemisches oder eines individuellen Diastereomeren, vorzugsweise des Isomeren B, sowie pharmazeutisch verwendbare Säureadditionssalze und Komplexe davon.

11. Pharmazeutische Präparate enthaltend mindestens eine der Verbindungen gemäss einem der Ansprüche 3, 6, 8 und 9.

12. Pharmazeutische Präparate enthaltend mindestens eine der Verbindungen gemäss einem der Ansprüche 4, 5, 7 und 10.

13. Verbindung gemäss einem der Ansprüche 3, 6, 8 und 9 als Mittel zur Behandlung von Blutverlusten im gastrointestinalen Trakt.

14. Verbindung gemäss einem der Ansprüche 4, 5, 7 und 10 als Mittel zur Behandlung von Blutverlusten im gastrointestinalen Trakt.

15. Verbindung gemäss einem der Ansprüche 3, 6, 8 und 9 als Antidiabeticum.

16. Verbindung gemäss einem der Ansprüche 4, 5, 7 und 10 als Antidiabeticum.

17. Eine Verbindung der Formel

└Asn–Phe–Phe–trp–Lys(A)–Thr(B)–

Phe–Gaba(Ar┘ (II)

worin trp für L–Trp oder D–Trp, in welchen der Benzolring durch ein Halogenatom substituiert sein kann, und Gaba(Ar) für den Rest einer durch ein cyclisches Hydrocarbyl substituierten γ-Aminobuttersäure steht, A eine ε-Aminoschutzgruppe oder Wasserstoff und B eine Hydroxylschutzgruppe oder Wasserstoff bedeutet, wobei nur eines der Symbole A und B für Wasserstoff stehen kann, sowie Säureadditionssalze davon.

**Patentansprüche für den Vertragsstaat: AT**

1. Verfahren zur Herstellung von cyclischen Octapeptiden der Formel

└Asn–Phe–Phe–trp–Lys–Thr–Phe–Gaba(Ar)┘ (I)

5 6 7 8 9 10 11 12

worin trp für L–Trp oder D–Trp, in welchen der Benzolring durch ein Halogenatom substituiert sein kann, und Gaba(Ar) für den Rest einer durch ein cyclisches Hydrocarbyl substituierten γ-Aminobuttersäure steht, sowie Säureadditionssalzen und Komplexen davon, dadurch gekennzeichnet, dass man ein lineares Peptid der Formel

H–[II′]–C (III)

worin II' einen der unten definierten Formel II entsprechenden Rest darstellt, in welchem die amidische Bindung zwischen zwei beliebigen benachbarten Aminosäureresten des Peptidringes unterbrochen ist, und C für eine freie Hydroxylgruppe, eine durch eine Aktivierungsgruppe abgewandelte Hydroxylgruppe oder die Hydrazinogruppe $-NH-NH_2$ steht, cyclisiert, und in einer erhaltenen Verbindung der Formel

└Asn–Phe–Phe–trp–Lys(A)–Thr(B)–

Phe–Gaba(Ar┘ (II)

worin Gaba(Ar) und trp die obgenannten Bedeutungen haben, A eine ε-Aminoschutzgruppe oder Wasserstoff und B eine Hydroxylschutzgruppe oder Wasserstoff bedeutet, wobei nur eines der Symbole A und B für Wasserstoff stehen kann, die Schutzgruppe(n) abspaltet, und, wenn erwünscht, aus einem erhaltenen Säureadditionssalz die entsprechende Base freisetzt und/oder eine erhaltene Base in ein Säureadditionssalz davon umwandelt.

2. Verfahren gemäss Anspruch 1, dadurch gekennzeichnet, dass man ein Octapeptid der Formel I, worin trp für L–Trp oder D–Trp und Gaba(Ar) für den Rest einer durch ein cyclisches Hydrocarbyl substituierten γ-Aminobuttersäure steht, oder ein Säureadditionssalz oder einen Komplex davon herstellt.

3. Verfahren gemäss Anspruch 1, dadurch gekennzeichnet, dass man eine Verbindung der Formel I, worin trp für D–Trp und Gaba(Ar) für den Rest der 4-Amino-3-cyclohexylbuttersäure, 4-Amino-3-phenylbuttersäure, 4-Amino-3-(1-naphthyl)-buttersäure oder 4-Amino-3-(2-naphthyl)-buttersäure steht, in Form eines Diastereomerengemisches oder eines individuellen Diastereomeren, insbesondere des Isomeren B, oder pharmazeutisch verwendbare Säureadditionssalze oder Komplexe davon, herstellt.

4. Verfahren gemäss Anspruch 1, dadurch gekennzeichnet, dass man eine Verbindung der Formel I, worin trp für D–Trp und Gaba(Ar) für den Rest der 4-Amino-3-(4-fluorphenyl)-buttersäure, 4-Amino-3-(4-nitrophenyl)-buttersäure, 4-Amino-3-(4-chlor-1-naphthyl)-buttersäure oder 4-Amino-3-(3-phenoxyphenyl)-buttersäure steht, in Form eines Diastereomerengemisches oder eines individuellen Diastereomeren, insbesondere des Isomeren B, oder pharmazeutisch verwendbare Säureadditionssalze oder Komplexe davon, herstellt.

5. Verfahren gemäss Anspruch 1, dadurch gekennzeichnet, dass man [D–Trp$^8$; β-Cyclohexyl-Gaba$^{12}$]-cyclo-somatostatin(5–12)-octapeptid in Form eines Diastereomerengemisches oder eines individuellen Diastereomeren, insbesondere des Isomeren B, oder parmazeutisch verwendbare Säureadditionssalze oder Komplexe davon, herstellt.

6. Verfahren gemäss Anspruch 1, dadurch gekennzeichnet, dass man [D–Trp$^8$; β-(3-Phenoxyphenyl)-Gaba$^{12}$]-cyclo-somatostatin(5–12)-octapeptid, in Form eines Diastereomerengemisches oder eines individuellen Diastereomeren, insbesondere des Isomeren B, oder pharmazeutisch verwendbare Säureadditionssalze oder Komplexe davon, herstellt.

7. Verfahren gemäss Anspruch 1, dadurch gekennzeichnet, dass man [D–Trp$^8$; β-(1-Naphthyl)-Gaba$^{12}$]-cyclo-somatostatin(5–12)-octapeptid, in Form eines Diastereomerengemisches oder eines individuellen Diastereomeren, insbesondere des Isomeren B, oder pharmazeutisch verwendbare Säureadditionssalze oder Komplexe davon, herstellt.

8. Verfahren zur Herstellung von pharmazeutischen Präparaten, enthaltend mindestens eine der gemäss Anspruch 1 erhältlichen Verbindungen der Formel I, dadurch gekennzeichnet, dass man mindestens eine dieser Verbindungen mit mindestens einem pharmazeutisch verwendbaren Hilfs- oder Trägermaterial vermischt.

9. Verfahren zur Herstellung von pharmazeutischen Präparaten, enthaltend mindestens eine der gemäss Anspruch 2 erhältlichen Verbindungen, dadurch gekennzeichnet, dass man mindestens eine dieser Verbindungen mit mindestens einem pharmazeutisch verwendbaren Hilfs- oder Trägermaterial vermischt.

10. Verfahren gemäss Anspruch 1, dadurch gekennzeichnet, dass man als Ausgangsstoff eine Verbindung der Formel

└Asn–Phe–Phe–trp–Lys(A)–Thr(B)–Phe–Gaba(Ar)┘ (II)

worin trp für L–Trp oder D–Trp, in welchen der Benzolring durch ein Halogenatom substituiert sein kann, und Gaba(Ar) für den Rest einer durch ein cyclisches Hydrocarbyl substituierten γ-Aminobuttersäure steht, A eine ε-Aminoschutzgruppe oder Wasserstoff und B eine Hydroxylschutzgruppe oder Wasserstoff bedeutet, wobei nur eines der Symbole A und B für Wasserstoff stehen kann, oder ein Säureadditionssalz davon, verwendet, die man durch die Cyclisierung eines linearen Peptids der Formel

H–Asn–Phe–Phe–trp–Lys(A)–Thr(B)–Phe–Gaba(-Ar)–C (IIIb)

worin trp und Gaba(Ar) die oben angegebenen Bedeutungen haben, A für eine ε-Aminoschutzgruppe oder Wasserstoff, B für eine Hydroxylschutzgruppe oder Wasserstoff, wobei nur eines der Symbole A und B für Wasserstoff stehen kann, und C für eine freie Hydroxylgruppe, eine durch eine Aktivierungsgruppe abgewandelte Hydroxylgruppe oder die Hydrazinogruppe $-NH-NH_2$ steht, herstellt.

**Claims for the Contracting States: BE, CH, DE, FR, GB, IT, LI, LU, NL, SE**

1. Process for the manufacture of cyclic octapeptides of the formula

└Asn–Phe–Phe–trp–Lys–Thr–Phe–Gaba(Ar)┘ (I)

5 6 7 8 9 10 11 12

in which

trp represents L–Trp or D–Trp, in which the benzene ring may be substituted by a halogen atom, and

Gaba(Ar) represents the residue of a γ-aminobutyric acid substituted by a cyclic hydrocarbyl radical,

and acid addition salts and complexes thereof, characterised in that a linear peptide of the formula

H–[II′]–C (III)

in which

II′ represents a radical corresponding to the formula II defined below in which the amide bond is interrupted between any two adjacent amino acid residues of the peptide ring, and

C represents a free hydroxyl group, a hydroxyl group modified by an activating group or represents the hydrazino group $-NH-NH_2$,

is cyclised and, in a resulting compound of the formula

Asn–Phe–Phe–trp–Lys(A)–Thr(B)–

Phe–Gaba(Ar) (II)

in which

Gaba(Ar) and trp have the meanings given above,

A represents an ε-amino-protecting group or hydrogen and

B represents a hydroxyl-protecting group or hydrogen,

it being possible for only one of the symbols A and B to represent hydrogen, the protecting group(s) is(are) split off and, if desired, the corresponding base is liberated from a resulting acid addition salt and/or a resulting base is converted into an acid addition salt thereof.

2. Process according to claim 1, characterised in that an octapeptide of the formula I, in which

trp represents L–Trp or D–Trp and

Gaba(Ar) represents the residue of a γ-aminobutyric acid substituted by a cyclic hydrocarbyl radical,

or an acid addition salt or a complex thereof is manufactured.

3. A cyclic octapeptide of the formula

Asn–Phe–Phe–trp–Lys–Thr–Phe–Gaba(Ar)

5 6 7 8 9 10 11 12 (I)

in which

trp represents L–Trp or D–Trp, in which the benzene ring may be substituted by a halogen atom, and

Gaba(Ar) represents the residue of a γ-aminobutyric acid substituted by a cyclic hydrocarbyl radical,

and also pharmaceutically acceptable acid addition salts and complexes thereof.

4. A compound according to claim 3 in which trp represents L–Trp or D–Trp and Gaba(Ar) represents the residue of a γ-aminobutyric acid substituted by a cyclic hydrocarbyl radical, and also pharmaceutically acceptable acid addition salts and complexes thereof.

5. A compound according to claim 4, in which trp represents D–Trp and Gaba(Ar) represents the residue of
4-amino-3-cyclohexylbutyric acid,
4-amino-3-phenylbutyricacid,
4-amino-3-(1-naphthyl)-butyric acid
or 4-amino-3-(2-naphthyl)-butyric acid, in the form of a diastereoisomeric mixture or of an individual diastereoisomer, and also pharmaceutically acceptable acid addition salts and complexes thereof.

6. A compound according to claim 3, in which trp represents D–Trp and Gaba(Ar) represents the residue of 4-amino-3-(4-fluorophenyl)-butyric acid,
4-amino-3-(4-nitrophenyl)-butyric acid,
4-amino-3-(4-chloro-1-naphthyl)-butyric acid or
4-amino-3-(3-phenoxyphenyl)-butyric acid,
in the form of a diastereoisomeric mixture or of an individual diastereoisomer, and also pharmaceutically acceptable acid addition salts and complexes thereof.

7. [D–Trp$^8$; β-cyclohexyl-Gaba$^{12}$]-cyclo-somatostatin(5–12)-octapeptide, in the form of a diastereoisomeric mixture or of an individual diastereoisomer, and also pharmaceutically acceptable acid addition salts and complexes thereof.

8. [D–Trp$^8$; β-(3-phenoxyphenyl)-Gaba$^{12}$]-cyclo-somatostatin(5–12)-octapeptide, in the form of a diastereoisomeric mixture of an individual diastereoisomer, and also pharmaceutically acceptable acid addition salts and complexes thereof.

9. A compound according to one of claims 3 to 8 in the form of the isomer B.

10. [D–Trp$^8$; β-(1-naphthyl)-Gaba$^{12}$]-cyclo-somatostatin(5–12)-octapeptide, in the form of a diastereoisomeric mixture or of an individual diastereoisomer, preferably of the isomer B, and also pharmaceutically acceptable acid solution salts and complexes thereof.

11. Pharmaceutical preparations containing at least one of the compounds according to one of claims 3, 6, 8 and 9.

12. Pharmaceutical preparations containing at least one of the compounds according to one of claims 4, 5, 7 and 10.

13. Compound according to one of claims 3, 6, 8 and 9 as an agent for the treatment of blood losses in the gastro-intestinal tract.

14. Compound according to one of claims 4, 5, 7 and 10 as agent for the treatment of blood losses in the gastro-intestinal tract.

15. Compound according to one of claims 3, 6, 8 and 9 as anti-diabetic.

16. Compound according to one of claims 4, 5, 7 and 10 as anti-diabetic.

17. A compound of the formula

Asn–Phe–Phe–trp–Lys(A)–Thr(B)–

Phe–Gaba(Ar) (II)

in which

trp represents L–Trp or D–Trp, in which the benzene ring may be substituted by a halogen atom, and

Gaba(Ar) represents the residue of a γ-aminobutyric acid substituted by a cyclic hydrocarbyl radical,

A represents an ε-amino-protecting group or hydrogen and

B represents a hydroxyl-protecting group or hydrogen,

it being possible for only one of the symbols A and B to represent hydrogen, and also acid addition salts thereof.

**Claims for the Contracting State: AT**

1. Process for the manufacture of cyclic octapeptides of the formula

Asn–Phe–Phe–trp–Lys–Thr–Phe–Gaba(Ar)

5 6 7 8 9 10 11 12 (I)

in which

trp represents L–Trp or D–Trp, in which the benzene ring may be substituted by a halogen atom, and

Gaba(Ar) represents the residue of a γ-aminobutyric acid substituted by a cyclic hydrocarbyl radical,

and acid addition salts and complexes thereof, characterised in that a linear peptide of the formula

H–[II′]–C (III)

in which

II′ represents a radical corresponding to the formula II defined below in which the amide bond is interrupted between any two adjacent amino acid residues of the peptide ring, and

C represents a free hydroxyl group, a hydroxyl group modified by an activating group or represents the hydrazino group $-NH-NH_2$,

is cyclised and, in a resulting compound of the formula

Asn–Phe–Phe–trp–Lys(A)–Thr(B)–

Phe–Gaba(Ar) (II)

in which

Gaba(Ar) and trp have the meanings given above,

A represents an ε-amino-protecting group or hydrogen and

B represents a hydroxyl-protecting group or hydrogen,

it being possible for only one of the symbols A and B to represent hydrogen, the protecting group(s) is(are) split off and, if desired, the corresponding base is liberated from a resulting acid addition salt and/or a resulting base is converted into an acid addition salt thereof.

2. Process according to claim 1, characterised in that an octapeptide of the formula I, in which

trp represents L–Trp or D–Trp and

Gaba(Ar) represents the residue of a γ-aminobutyric acid substituted by a cyclic hydrocarbyl radical,

or an acid addition salt or a complex thereof is manufactured.

3. Process according to claim 1, characterised in that a compound of the formula 1, in which trp represents D–Trp and Gaba(Ar) represents the residue of 4-amino-3-cyclohexylbutyric acid, 4-amino-3-phenylbutyric acid, 4-amino-3-(1-naphthyl)-butyric acid or 4-amino-3-(2-naphthyl)-butyric acid, in the form of a diastereoisomeric mixture or of an individual diastereoisomer, particularly of the isomer B, or pharmaceutically acceptable acid solution salts and complexes thereof are manufactured.

4. Process according to claim 1, characterised in that a compound of the formula I, in which trp represents D–Trp and Gaba(Ar) represents the residue of

4-amino-3-(4-fluorophenyl)-butyric acid,

4-amino-3-(4-nitrophenyl)-butyric acid,

4-amino-3-(4-chloro-1-naphthyl)-butyric acid

or 4-amino-3-(3-phenoxyphenyl)-butyric acid, in the form of a diastereoisomeric mixture or of an individual diastereoisomer, particularly of the isomer B, or pharmaceutically acceptable acid addition salts and complexes thereof are manufactured.

5. Process according to claim 1, characterised in that

[D–Trp$^{8}$; β-cyclohexyl-Gaba$^{12}$]-cyclosomatostatin-(5–12)-octapeptide,

in the form of a diastereoisomeric mixture or of an individual diastereoisomer, particularly of the isomer B, or pharmaceutically acceptable acid addition salts and complexes thereof are manufactured.

6. Process according to claim 1, characterised in that

[D–Trp$^{8}$; β-(3-phenoxyphenyl)-Gaba$^{12}$]-cyclosomatostatin(5–12)-octapeptide,

in the form of a diastereoisomeric mixture or of an individual diastereoisomer, particularly of the isomer B, or pharmaceutically acceptable acid addition salts and complexes thereof are manufactured.

7. Process according to claim 1, characterised in that

[D–Trp$^{8}$; β-(1-naphthyl)-Gaba$^{12}$]-cyclosomatostatin(5–12)-octapeptide,

in the form of a diastereoisomeric mixture or of an individual diastereoisomer, particularly of the isomer B, or pharmaceutically acceptable acid addition salts or complexes thereof are manufactured.

8. Process for the manufacture of pharmaceutical preparations containing at least one of the compounds of the formula I obtainable according to claim 1, characterised in that at least one of these compounds is mixed with at least one pharmaceutically acceptable adjunct or carrier material.

9. Process for the manufacture of pharmaceutical preparations containing at least one of the compounds obtainable according to claim 2, characterised in that at least one of these compounds is mixed with at least one pharmaceutically acceptable adjunct or carrier material.

10. Process according to claim 1, characterised in that there is used as starting material a compound of the formula

└Asn–Phe–Phe–trp–Lys(A)–Thr(B)–Phe–Gaba(Ar)┘ (II)

in which trp represents L–Trp or D–Trp, in which the benzene ring can be substituted by a halogen atom, and Gaba(Ar) represents the residue of a γ-aminobutyric acid substituted by a cyclic hydrocarbyl radical, A represents an ε-amino-protecting group or hydrogen, and B represents a hydroxyl-protecting group or hydrogen, it being possible for only one of the symbols A and B to represent hydrogen, or an acid addition salt thereof, which compound is manufactured by cyclisation of a linear peptide of the formula

H–Asn–Phe–Phe–trp–Lys(A)–Thr(B)–Phe–Gaba(Ar)–C (IIIb)

in which trp and Gaba(Ar) have the meanings defined above, A represents an ε-amino-protecting group or hydrogen, B represents a hydroxyl-protecting group or hydrogen, it being possible for only one of the symbols A and B to represent hydrogen, and C represents a free hydroxyl group, a hydroxyl group modified by an activating group or represents the hydrazino group $-NH-NH_2$.

**Revendications pour les Etats contractants: BE, CH, DE, FR, GB, IT, LI, LU, NL, SE**

1. Procédé de préparation d'octapeptides cycliques de formule

└Asn–Phe–Phe–trp–Lys–Thr–Phe–Gaba(Ar)┘

5 6 7 8 9 10 11 12 (I)

dans laquelle trp représente L–Trp ou D–Trp, dans lesquels le noyau benzénique peut être substitué par un atome d'halogène, et Gaba(Ar) représente le reste d'un acide gamma-aminobutyrique portant un substituant hydrocarboné cyclique, de leurs sels formés par addition avec des acides et de leurs complexes, caractérisé en ce qu'on cyclise un peptide linéaire de formule

H–[II′]–C (III)

dans laquelle II′ représente l'un des restes correspondant à la formule II définie ci-dessous, dans lequel la liaison amide entre deux restes d'aminoacides voisins quelconques du cycle peptidique est interrompue, et C représente un groupe hydroxy libre, un groupe hydroxy modifié par un groupe activant ou le groupe hydrazino $-NH-NH_2$, et dans un composé obtenu, de formule

└Asn–Phe–Phe–trp–Lys(A)–Thr(B)–Phe–Gaba(Ar)┘ (II)

dans laquelle Gaba(Ar) et trp ont les significations indiquées ci-dessus, A représente un groupe protecteur du groupe epsilon-amino ou l'hydrogène et B un groupe protecteur du groupe hydroxy ou l'hydrogène, un seul des symboles A et B pouvant représenter l'hydrogène, on élimine le ou les groupes protecteurs et, si on le désire, on libère la base correspondante à partir d'un sel d'acide obtenu et/ou on convertit une base obtenue en un sel formé par addition avec un acide.

2. Procédé selon la revendication 1, caractérisé en ce que l'on prépare un octapeptide de formule I dans laquelle trp représente l–Trp ou D–Trp et Gaba(Ar) représente le reste d'un acide gamma-aminobutyrique portant un substituant hydrocarboné cyclique, ou un sel d'un tel composé formé par addition avec un acide ou un complexe d'un tel composé.

3. Un octapeptide cyclique de formule

└Asn–Phe–Phe–trp–Lys–Thr–Phe–Gaba(Ar)┘ (I)

5 6 7 8 9 10 11 12

dans laquelle trp représente L–Trp ou D–Trp, dans lesquels le noyau benzénique peut être substitué par un atome d'halogène, et Gaba(Ar) représente le reste d'un acide gamma-aminobutyrique portant un substituant hydrocarboné cyclique, et ses sels formés par addition avec des acides et complexes acceptables pour l'usage pharmaceutique.

4. Un composé selon la revendication 3, dans lequel trp représente L–Trp ou D–Trp et Gaba(Ar) représente le reste d'un acide gamma-aminobutyrique portant un substituant hydrocarboné cyclique, et ses sels formés par addition avec des acides et complexes acceptables pour l'usage pharmaceutique.

5. Un composé selon la revendication 4, dans lequel trp représente D–Trp et Gaba(Ar) représente le reste de l'acide 4-amino-3-cyclohexylbutyrique, de l'acide 4-amino-3-phénylbutyrique, de l'acide 4-amino-3-(1-naphtyl)-butyrique ou de l'acide 4-amino-3-(2-naphtyl)-butyrique, à l'état d'un mélange de diastéréoisomères ou d'un diastéréoisomère individuel, et ses sels formés par addition avec des acides et complexes acceptables pour l'usage pharmaceutique.

6. Un composé selon la revendication 3, dans lequel trp représente D–Trp et Gaba(Ar) représente le reste de l'acide
4-amino-3-(4-fluorophényl)-butyrique, de l'acide
4-amino-3-(4-nitrophényl)-butyrique, de l'acide
4-amino-3-(4-chloro-1-naphtyl)-butyrique ou de l'acide
4-amino-3-(3-phénoxyphényl)-butyrique,
à l'état d'un mélange de diastéréoisomères ou

d'un diastéréoisomère individuel, ses sels formés par addition avec des acides et complexes acceptables pour l'usage pharmaceutique.

7. L'octapeptide [D–Trp$^8$; β-Cyclohexyl-Gaba$^{12}$]-cyclo-somatostatine-(5–12), à l'état de mélange de diastéréoisomères ou d'un diastéréoisomère individuel, ses sels formés par addition avec des acides et complexes acceptables pour l'usage pharmaceutique.

8. L'octapeptide [D–Trp$^8$; β-(3-Phénoxyphényl)-Gaba$^{12}$]-cyclosomatostatine-(5–12) à l'état de mélange de diastéréoisomères ou de diastéréoisomère individuel, ses sels formés par addition avec des acides et complexes acceptables pour l'usage pharmaceutique.

9. Un composé selon l'une des revendications 3 à 8 à l'état de l'isomère B.

10. L'octapeptide [D–Trp$^8$; β-(1-Naphtyl)-Gaba$^{12}$]-cyclo-somato-statine-(5–12) à l'état de mélange de diastéréoisomères ou d'un diastéréoisoemère individuel, de préférence de l'isomère B, et ses sels formés par addition avec des acides et complexes acceptables pour l'usage pharmaceutique.

11. Compositions pharmaceutiques contenant au moins un des composés selon l'une des revendications 3, 6, 8 et 9.

12. Compositions pharmaceutiques contenant au moins un des composés selon l'une des revendications 4, 5, 7 et 10.

13. Composé selon l'une des revendications 3, 6, 8 et 9, en tant qu'agent pour le traitement des pertes de sang dans la voie gastro-intestinale.

14. Composé selon l'une des revendications 4, 5, 7 et 10, en tant qu'agent pour le traitement des pertes de sang dans la voie gastro-intestinale.

15. Composé selon l'une des revendications 3, 6, 8 et 9, en tant qu'antidiabétique.

16. Composé selon l'une des revendications 4, 5, 7 et 10 en tant qu'antidiabétique.

17. Un composé de formule

└Asn–Phe–Phe–trp–Lys(A)–Thr(B)–
Phe–Gaba(Ar)┘ (II)

dans laquelle trp représente L–Trp ou D–Trp, dans lesquels le noyau benzénique peut être substitué par un atome d'halogène, et Gaba(Ar) représente le reste d'un acide gamma-aminobutyrique portant un substituant hydrocarboné cyclique, A représente un groupe protecteur du groupe epsilon-amino ou l'hydrogène et B un groupe protecteur du groupe hydroxy ou l'hydrogène, un seul des symboles A et B pouvant représenter l'hydrogène, et ses sels formés par addition avec des acides.

**Revendications pour l'Etat contractant: AT**

1. Procédé de préparation d'octapeptides cycliques de formule

└Asn–Phe–Phe–trp–Lys–Thr–Phe–Gaba(Ar)┘ (I)
5 6 7 8 9 10 11 12

dans laquelle trp représente L–Trp ou D–Trp, dans lesquels le noyau benzénique peut être substitué par un atome d'halogène, et Gaba(Ar) représente le reste d'un acide gamma-aminobutyrique portant un substituant hydrocarboné cyclique, et de leurs sels formés par addition avec des acides et complexes, caractérisé en ce que l'on cyclise un peptide linéaire de formule

H–[II']–C (III)

dans laquelle II' représente un reste répondant à la formule II définie ci-dessous, dans lequel la liaison amide entre deux restes d'aminoacides quelconques voisins du cycle peptide est interrompue, et C représente un groupe hydroxy libre ou un groupe hydroxy modifié par un groupe activant ou le groupe hydrazino $-NH-NH_2$, et dans un composé obtenu, de formule

└Asn–Phe–Phe–trp–Lys(A)–Thr(B)–
Phe–Gaba(Ar)┘ (II)

dans laquelle Gaba(Ar) et trp ont les significations indiquées ci-dessus, A représente un groupe protecteur du groupe epsilon-amino ou l'hydrogène et B un groupe protecteur d'un groupe hydroxy ou l'hydrogène, un seul des symboles A et B pouvant représenter l'hydrogène, on élimine le ou les groupes protecteurs, et si on le désire, on libère la base correspondante à partir d'un sel d'acide obtenu et/ou on convertit une base obtenue en un sel par addition avec un acide.

2. Procédé selon la revendication 1, caractérisé en ce que l'on prépare un octapeptide de formule I dans laquelle trp représente L–Trp ou D–Trp et Gaba(Ar) représente le reste d'un acide gamma-aminobutyrique portant un substituant hydrocarboné cyclique, ou un sel d'un tel composé formé par addition avec un acide ou un complexe d'un tel composé.

3. Procédé selon la revendication 1, caractérisé en ce que l'on prépare un composé de formule I dans laquelle trp représente D–Trp et Gaba(Ar) représente le reste de l'acide 4-amino-3-cyclohexylbutyrique, de l'acide 4-amino-3-phénylbutyrique, de l'acide 4-amino-4-(1-naphtyl)-butyrique ou de l'acide 4-amino-3-(2-naphtyl)-butyrique, à l'état d'un mélange de diastéréoisomères ou d'un diastéréoisomère individuel, en particulier de l'isomère B, ou des sels de ce composé formés par addition avec des acides ou des complexes de ce composé acceptables pour l'usage pharmaceutique.

4. Procédé selon la revendication 1, caractérisé en ce que l'on prépare un composé de formule I dans laquelle trp représente D–Trp et Gaba(Ar) représente le reste de l'acide
4-amino-3-(4-fluorphényl)-butyrique, de l'acide
4-amino-3-(4-nitrophényl)-butyrique, de l'acide
4-amino-3-(4-chloro-1-naphtyl)-butyrique ou de l'acide
4-amino-3-(3-phénoxyphényl)-butyrique
à l'état d'un mélange de diastéréoisomères ou d'un diastéréoisomère individuel, en particulier

de l'isomère B, ou des sels de ce composé formés par addition avec des acides ou des complexes de ce composé acceptables pour l'usage pharmaceutique.

5. Procédé selon la revendication 1, caractérisé en ce que l'on prépare l'octapeptide

[D–Trp$^{8}$; β-Cyclohexyl-Gaba$^{12}$]-cyclo-somatostatine-(5–12)

à l'état d'un mélange de diastéréoisomères ou d'un diastéréoisomère individuel, en particulier de l'isomère B, ou des sels formés par addition avec des acides ou des complexes de ce composé acceptables pour l'usage pharmaceutique.

6. Procédé selon la revendication 1, caractérisé en ce que l'on prépare l'octapeptide

[D–Trp$^{8}$; β-(3-Phénoxyphényl)-Gaba$^{12}$] -cyclo-somatostatine-(5–12)

à l'état d'un mélange de diastéréoisomères ou d'un diastéréoisomère individuel, en particulier de l'isomère B, ou des sels formés par addition avec des acides ou complexes de ce composé acceptables pour l'usage pharmaceutique.

7. Procédé selon la revendication 1, caractérisé en ce que l'on prépare l'octapeptide

[D–Trp$^{8}$; β-(1-Naphtyl)-Gaba$^{12}$]-cyclo-somatostatine-(5–12),

à l'état d'un mélange de diastéréoisomères ou d'un diastéréoisomère individuel, en particulier de l'isomère B, ou des sels formés par addition avec des acides ou complexes de ce composé acceptables pour l'usage pharmaceutique.

8. Procédé de préparation de compositions pharmaceutiques contenant au moins l'un des composés de formule I obtenus selon la revendication 1, caractérisé en ce que l'on mélange au moins un de ces composés avec au moins un produit auxiliaire ou véhicule acceptable pour l'usage pharmaceutique.

9. Procédé de préparation de compositions pharmaceutiques contenant au moins un des composés obtenus selon la revendication 2, caractérisé en ce que l'on mélange au moins un de ces composés avec au moins un produit auxiliaire ou véhicule acceptable pour l'usage pharmaceutique.

10. Procédé selon la revendication 1, caractérisé en ce que l'on utilise en tant que produit de départ un composé de formule

Asn–Phe–Phe–trp–Lys(A)–Thr(B)–

Phe–Gaba(Ar) (II)

dans laquelle trp représente L–Trp ou D–Trp, dans lesquels le noyau benzénique peut être substitué par un atome d'halogène, et Gaba(Ar) représente le reste d'un acide gamma-aminobutyrique portant un substituant hydrocarboné cyclique, A est un groupe protecteur d'un groupe epsilon-amino ou l'hydrogène et B un groupe protecteur d'un groupe hydroxy ou l'hydrogène, un seul des symboles A et B pouvant représenter l'hydrogène, ou un sel d'un tel composé formé par addition avec un acide, qu'on prépare par cyclisation d'un peptide linéaire de formule

H–Asn–Phe–Phe–trp–Lys(A)–Thr(B)–

Phe–Gaba(Ar)–C (IIIb)

dans laquelle trp et Gaba(Ar) ont les significations indiquées ci-dessus, A représente un groupe protecteur d'un groupe epsilon-amino ou l'hydrogène, B un groupe protecteur d'un groupe hydroxy ou l'hydrogène, un seul des symboles A et B pouvant représenter l'hydrogène, et C représente un groupe hydroxy libre, un groupe hydroxy modifié par un groupe activant ou le groupe hydrazino $-NH-NH_2$.